(19) Europäisches Patentamt
European Patent Office
Office européen des brevets

(11) Publication number: 0 502 575 A1

(12) **EUROPEAN PATENT APPLICATION**

(21) Application number: 92200563.2

(22) Date of filing: 27.02.92

(51) Int. Cl.5: C07D 217/24, C07D 401/10, C07F 9/62, A61K 31/47, A61K 31/675

(30) Priority: 06.03.91 US 665491
11.02.92 US 830621

(43) Date of publication of application:
09.09.92 Bulletin 92/37

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Patchett, Arthur A.
1090 Minisink Way
Westfield, NJ 07090(US)
Inventor: de Laszlo, Stephen E.
178 Monmouth Avenue
Atlantic Highlands, NJ 07716(US)
Inventor: Greenlee, William J.
115 Herrick Avenue
Teaneck, NJ 07666(US)

(74) Representative: Thompson, John Dr. et al
Merck & Co., Inc. European Patent
Department Terlings Park Eastwick Road
Harlow, Essex CM20 2OR(GB)

(54) **Substituted 1-(2H)-isoquinolinones.**

(57) Substituted 1-(2H)-isoquinolinones of the structural formula:

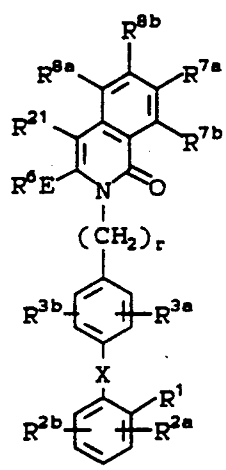

(I)

are angiotensin II antagonists which are useful in the treatment of hypertension and congestive heart failure.

EP 0 502 575 A1

## SUMMARY OF THE INVENTION

This invention relates to novel compounds of structural formula I which are angiotensin II antagonists useful in the treatment of hypertension, congestive heart failure, and elevated intraocular pressure.

It also relates to processes for preparing the novel compounds; pharmaceutical formulations comprising one or more of the compounds as active ingredient; and, a method of treatment of hypertension, congestive heart failure, and elevated intraocular pressure.

The compounds of this invention also have central nervous system (CNS) activity. They are useful in the treatment of cognitive dysfunctions including Alzheimer's disease, amnesia and senile dementia. These compounds also have anxiolytic and antidepressant properties and are therefore, useful in the relief of symptoms of anxiety and tension and in the treatment of patients with depressed or dysphoric mental states.

In addition, these compounds exhibit antidopaminergic properties and are thus useful to treat disorders that involve dopamine dysfunction such as schizophrenia. The compounds of this invention are especially useful in the treatment of these conditions in patients who are also hypertensive or have a congestive heart failure condition.

## BACKGROUND OF THE INVENTION

Renin-angiotensin system (RAS) plays a central role in the regulation of normal blood pressure and seems to be critically involved in hypertension development and maintenance as well as congestive heart failure. Angiotensin II (AII), an octapeptide hormone is produced mainly in the blood during the cleavage of angiotensin I by angiotensin converting enzyme (ACE) localized on the endothelium of blood vessels of lung, kidney, and many other organs, and is the end product of the RAS. AII is a powerful arterial vasoconstricter that exerts its action by interacting with specific receptors present on cell membranes. One of the possible modes of controlling the RAS is angiotensin II receptor antagonism. Several peptide analogs of A II are known to inhibit the effect of this hormone by competitively blocking the receptors, but their experimental and clinical applications have been limited by the partial agonist activity and lack of oral absorption [M. Antonaccio. Clin. Exp. Hypertens. A4, 27-46 (1982); D. H. P. Streeten and G. H. Anderson, Jr. - Handbook of Hypertension, Clinical Pharmacology of Antihypertensive Drugs, ed. A. E. Doyle, Vol. 5, pp. 246-271, Elsevier Science Publisher, Amsterdam, The Netherlands, 1984].

Recently, several non-peptide compounds have been described as A II antagonists. Illustrative of such compounds are those disclosed in U.S. Patents 4,207,324; 4,340,598; 4,576,958; 4,582,847; and 4,880,804; in European Patent Applications 028,834; 245,637; 253,310; and 291,969; and in articles by A.T. Chiu, et al. [Eur. J. Pharm. Exp. Therap, 157, 13-21 (1988)] and by P.C. Wong, et al. [J. Pharm. Exp. Therap, 247, 1-7-(1988)]. All of the U.S. Patents, European Patent Applications 028,834 and 253,310 and the two articles disclose substituted imidazole compounds which are generally bonded through a lower alkyl bridge to a substituted phenyl. European Patent Application 245,637 discloses derivatives of 4,5,6,7-tetrahydro-2H-imidazo[4,5-c]-pyridine-6-carboxylic acid and analogs thereof as antihypertensive agents.

None of the compounds disclosed in the above identified U.S. Patents, European Applications and articles have the heterobicyclic structure of the compounds of this invention.

## DETAILED DESCRIPTION OF THE INVENTION

This invention relates to substituted 1-(2H)-isoquinolinones of the formula I shown below which are angiotensin II antagonists and are useful in the treatment of hypertension, congestive heart failure, and elevated intraocular pressure.

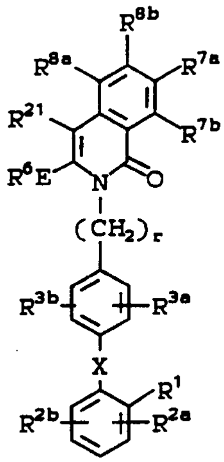

(I)

wherein:

$R^1$ is

(a) $-CO_2R^4$,
(b) $-SO_3R^5$,
(c) $-NHSO_2R^{22}$,
(d) $-PO(OR^5)_2$,
(e) $-SO_2-NH-R^{22}$,
(f)

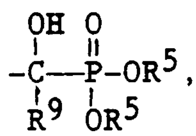

(g)

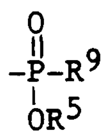

(h) $-SO_2NH-CO-R^{22}$,
(i) $-CH_2SO_2NH-CO-R^{22}$,
(j) $-CONH-SO_2R^{22}$,
(k) $-CH_2CONH-SO_2R^{22}$,
(l) $-NHSO_2NHCO-R^{22}$,
(m) $-NHCONHSO_2-R^{22}$,
(n)

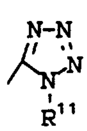

or

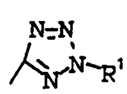

,

(o)

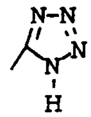 ,

(p)

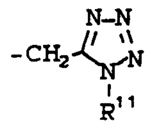 or 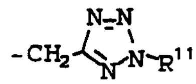,

(q)

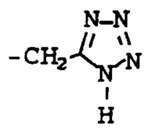 ,

(r)

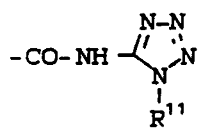 or 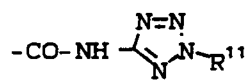,

(s)

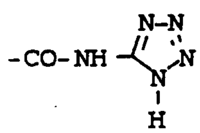 ,

(t) $-CONHNHSO_2CF_3$ ,
(u) $-SO_2NH-CN$,
(v)

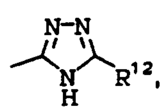,

(w)

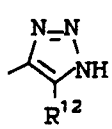,

(x) $-PO(OR^5)(OR^4)$,
(y) $-SO_2NHCONR^4R^{22}$, or
(z) $-CH_2SO_2NHR^{22}$;

wherein heteroaryl is an unsubstituted, monosubstituted or disubstituted five or six membered aromatic ring which contains from 1 to 3 heteroatoms selected from the group consisting of O, N or S and wherein the substituents are members selected from the group consisting of -OH, -SH, -$C_1$-$C_4$-alkyl, -$C_1$-$C_4$-alkoxy, -$CF_3$, Cl, Br, F, I, -$NO_2$, -$CO_2H$, -$CO_2$-($C_1$-$C_4$-alkyl), -$NH_2$, -NH($C_1$-$C_4$-alkyl) and -N($C_1$-$C_4$-alkyl)$_2$;

$R^{2a}$ and $R^{2b}$ are each independently
(a) H,
(b) Cl, Br, I, or F,
(c) $NO_2$,
(d) $NH_2$,
(e) $C_1$-$C_4$-alkylamino,
(f) di($C_1$-$C_4$-alkyl)amino,
(g) $SO_2NHR^9$,
(h) $CF_3$,
(i) $C_1$-$C_6$-alkyl,
(j) $C_1$-$C_6$-alkoxy,
(k) $C_1$-$C_6$-alkyl-S-,
(l) $C_2$-$C_6$-alkenyl,
(m) $C_2$-$C_6$-alkynyl;
(n) aryl,
(o) aryl($C_1$-$C_4$-alkyl), or
(p) $C_3$-$C_7$-cycloalkyl;

$R^{3a}$ is
(a) H,
(b) Cl, Br, I, or F,
(c) $C_1$-$C_6$-alkyl,
(d) $C_1$-$C_6$-alkoxy, or
(e) $C_1$-$C_6$-alkoxyalkyl;

$R^{3b}$ is
(a) H,
(b) Cl, Br, I, or F,
(c) $NO_2$,
(d) $C_1$-$C_6$-alkyl,
(e) $C_1$-$C_6$-acyloxy,
(f) $C_3$-$C_7$-cycloalkyl,
(g) $C_1$-$C_6$-alkoxy,
(h) -$NHSO_2R^4$,
(i) hydroxy($C_1$-$C_4$-alkyl),
(j) aryl($C_1$-$C_4$-alkyl),
(k) $C_1$-$C_4$-alkylthio,
(l) $C_1$-$C_4$-alkyl sulfinyl,
(m) $C_1$-$C_4$-alkyl sulfonyl,
(n) $NH_2$,
(o) $C_1$-$C_4$-alkylamino,
(p) di($C_1$-$C_4$-alkyl)amino,
(q) fluoro-$C_1$-$C_4$-alkyl-,
(r) -$SO_2$-$NHR^9$,
(s) aryl,
(t) furyl,
(u) $CF_3$,
(v) $C_2$-$C_6$-alkenyl, or
(w) $C_2$-$C_6$-alkynyl;

wherein aryl is phenyl or naphthyl, or a substituted phenyl or naphthyl with one or two substituents selected from the group consisting of Cl, Br, I, F, N-($R^4$)$_2$, $CO_2R^4$, $C_1$-$C_4$-alkyl, $C_1$-$C_4$-alkoxy, $NO_2$, $CF_3$, $C_1$-$C_4$-alkylthio, OH, or $SO_x$($C_1$-$C_4$-alkyl);

$R^4$ is H, aryl, $C_1$-$C_6$ alkyl, or substituted $C_1$-$C_6$ alkyl in which substituent is aryl or heteroaryl;

$R^{4a}$ is aryl, $C_1$-$C_6$-alkyl or a substituted $C_1$-$C_6$-alkyl with an aryl substituent;

$R^5$ is H,

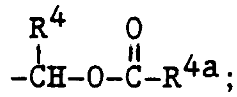

provided that when $R^4$ is H, $R^5$ is not H;

E is a single bond, $-NR^{13}(CH_2)_s-$, $-S(O)x(CH_2)_s-$ where s is 0 to 5, or CO-;

$R^6$ is

(a) aryl, or a substituted aryl with 1 or 2 substituents selected from the group consisting of Cl, Br, I, F, $-0-C_1-C_4$-alkyl, $C_1$-$C_4$-alkyl, $-NO_2$, $-CF_3$, $-SO_2NR^9R^{10}$, $-S-C_1-C_4$-alkyl, -OH, $-NH_2$, $C_3$-$C_7$-cycloalkyl, or $C_3$-$C_{10}$-alkenyl,
(b) $C_1$-$C_6$-alkyl, $C_2$-$C_5$-alkenyl or $C_2$-$C_5$-alkynyl, or a substituted $C_1$-$C_6$-alkyl, a substituted $C_2$-$C_5$-alkenyl or a substitued $C_2$-$C_5$-alkynyl, in which the substituent is selected from the group consisting of aryl, $C_3$-$C_7$-cycloalkyl, Cl, Br, I, F, $CF_3$, $CF_2CF_3$, $-NH_2$, $-NH(C_1-C_4\text{-alkyl})$, $-OR^4$ -N-($C_1$-$C_4$-alkyl)$_2$, $-NH-SO_2R^4$, $-COOR^4$, or $-SO_2NHR^9$,
(c) an unsubstituted, monosubstituted or disubstituted heteroaromatic 5 or 6 membered cyclic ring which contains one to three members selected from the group consisting of N, O, S, and wherein the substituents are members selected from the group consisting of -OH, -SH, $C_1$-$C_4$-alkyl, $C_1$-$C_4$-alkoxy, $-CF_3$, Cl, Br, I, F, or $NO_2$
(d) $C_3$-$C_7$-cycloalkyl,
(e) perfluoro-$C_1$-$C_4$-alkyl, or
(f) H;

$R^{7a}$, $R^{7b}$, $R^{8a}$ and $R^{8b}$ are independently

(a) H,
(b) $C_1$-$C_8$-alkyl or a substituted $C_1$-$C_8$-alkyl with a substituent selected from the group consisting of -OH, -guanidino, $C_1$-$C_4$-alkoxy, $-N(R^4)_2$, $COOR^4$, $-CON(R^4)_2$, $-O-COR^4$, -aryl, -heteroaryl, $-S(O)_x-R^{22}$, -tetrazol-5-yl, $-CONHSO_2R^{22}$, $-SO_2NH$-heteroaryl, $-SO_2NHCOR^{22}$, $-PO(OR^4)_2$, $-PO(OR^4)R^9$, $-SO_2NH-CN$, $-NR^{10}COOR^{22}$, $-(CH_2)_{1-4}R^4$, $-CO-R^4$, -CO-heteroaryl, $-NR^4CONR^4R^{22}$, $-NR^4COR^{22}$,

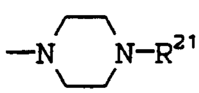 , 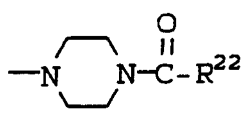 ,

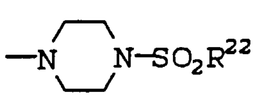 and 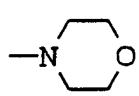 ,

(c) $-C_3$-$C_7$-cycloalkyl,
(d) aryl-$C_1$-$C_6$alkyl in which the aryl group is unsubstituted, mono or disubstituted with V or W,
(e) aryl or substituted aryl in which the substituents are V or W,
(f) Cl, Br, I, or F,
(g) $-OR^{22a}$,
(h) $-C_1$-$C_4$-perfluoroalkyl,

(i) $-S(O)_x-R^{22}$,
(j) $-COOR^4$,
(k) $-SO_3H$,
(l) $-NR^4R^{22}$,
(m) $-NR^{22a}COR^{22}$,
(n) $-NR^{22a}COOR^{22}$,
(o) $-SO_2NR^4R^9$,
(p) $-NO_2$,
(q) $-N(R^{22a})SO_2R^{22}$,
(r) $-NR^{22a}CONR^4R^{22}$,
(s)

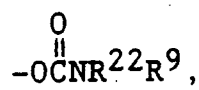

(t) $-NHSO_2R^{22}$,
(u) $-SO_2NH$-heteroaryl,
(v) $-SO_2NHCOR^{22}$,
(w) $-CONHSO_2R^{22}$,
(z) $-PO(OR^4)_2$,
(y) $-PO(OR^4)R^4$,
(z) -tetrazol-5-yl,
(aa) -CONH(tetrazol-5-yl),
(bb) $-COR^{22a}$,
(cc) $-SO_2NHCN$
(dd) $-NR^{22a}SO_2NR^4R^{22}$,
(ee) $-NR^{22a}SO_2OR^{22}$
(ff) $-CONR^4R^{22}$,
(gg)

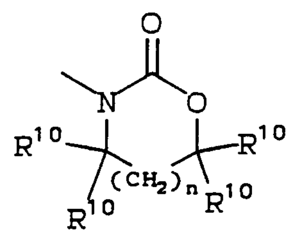

where n = 0 or 1,
(hh)

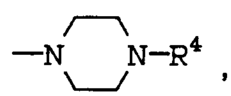

(ii)

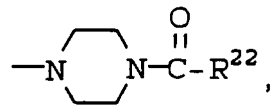

(jj)

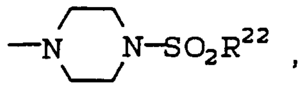

(kk)

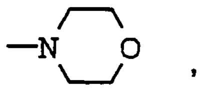

(ll) heteroaryl as defined hereinabove, or
(mm)

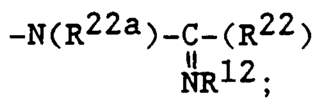

V and W are independently:
(a) hydrogen,
(b) $C_1$-$C_5$-alkoxy,
(c) $C_1$-$C_5$-alkyl,
(d) hydroxy,
(e) $C_1$-$C_5$-alkyl-$S(O)_x$-,
(f) CN,
(g) $NO_2$,
(h) $N(R^4)_2$,
(i) $CON(R^4)_2$,
(j) $CO_2R^4$,
(k) $COR^4$,
(l) $CF_3$,
(m) Cl, Br, I, or F,
(n) hydroxy-$C_1$-$C_5$-alkyl,
(o) $C_1$-$C_5$-alkylthio,
(p) -$SO_2NR^9R^{10}$,
(q) $C_3$-$C_7$-cycloalkyl, or
(r) $C_2$-$C_{10}$-alkenyl;

$R^9$ is H, $C_1$-$C_5$-alkyl, aryl or arylmethyl;
$R^{10}$ is H, $C_1$-$C_4$-alkyl;
$R^{11}$ is H, $C_1$-$C_6$-alkyl, $C_1$-$C_4$-alkenyl, $C_1$-$C_4$-alkoxy alkyl, or

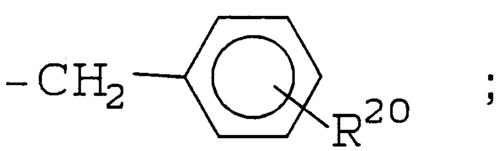

$R^{12}$ is -CN, -$NO_2$, -$CF_3$ or -$CO_2R^4$;
$R^{13}$ is H, ($C_1$-$C_4$-alkyl)CO-, $C_1$-$C_6$-alkyl, allyl, $C_3$-$C_6$-cycloalkyl, aryl or arylmethyl;
$R^{14}$ is H, $C_1$-$C_8$-alkyl, $C_1$-$C_8$-perfluoroalkyl, $C_3$-$C_6$-cycloalkyl, aryl or arylmethyl;
$R^{15}$ is H, $C_1$-$C_6$-alkyl;
$R^{16}$ is H, $C_1$-$C_6$-alkyl, $C_3$-$C_6$-cycloalkyl, aryl or arylmethyl;
$R^{17}$ is -$NR^9R^{10}$, -$OR^{10}$, -$NHCONH_2$, -$NHCSNH_2$,

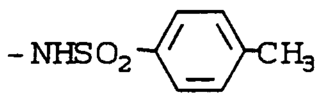 or 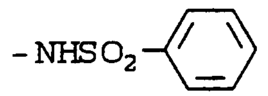 ;

$R^{18}$ and $R^{19}$ are independently $C_1$-$C_4$-alkyl or taken together are $-(CH_2)_q-$ where q is 2 or 3;

$R^{20}$ is H, $-NO_2$, $-NH_2$, -OH or $-OCH_3$;

$R^{21}$ is

(a) H,
(b) Cl, F, Br or I,
(c) aryl,
(d) heteroaryl, or
(e) $C_1$-$C_4$-alkyl or a substituted $C_1$-$C_4$-alkyl with a substituent selected from the group consisting of aryl, heteroaryl, -OH, $-NH_2$, -NH($C_1$-$C_4$-alkyl),-N($C_1$-$C_4$-alkyl$)_2$, $-CO_2R^{4a}$, Cl, Br, F, I, or $-CF_3$;

$R^{22}$ is

(a) aryl,
(b) heteroaryl,
(c) $C_3$-$C_7$-cycloalkyl,
(d) $C_1$-$C_6$-alkyl or a substituted $C_1$-$C_6$-alkyl with one or two substituents selected from the group consisting of aryl, heteroaryl, -OH, -SH, $C_1$-$C_4$-alkyl, -O($C_1$-$C_4$-alkyl), -S($C_1$-$C_4$-alkyl), $-CF_3$, Cl, Br, F, I, $-NO_2$, $-CO_2H$, $CO_2$-($C_1$-$C_4$-alkyl), $-NH_2$, -NH($C_1$-$C_4$-alkyl), -N($C_1$-$C_4$-alkyl$)_2$, $-PO_3H_2$, -PO(OH)(O-$C_1$-$C_4$-alkyl), $-PO(OR^4)R^9$, morpholinyl, $-SO_xR^4$ piperazinyl-4-$COR^{22}$ or $C_1$-$C_4$ alkylpiperazinyl, or
(e) perfluoro-$C_1$-$C_4$-alkyl;

$R^{22a}$ is

(a) hydrogen,
(b) aryl,
(c) heteroaryl,
(d) $C_3$-$C_7$-cycloalkyl,
(e) $C_1$-$C_6$-alkyl or a substituted $C_1$-$C_6$-alkyl with a substituent selected from the group consisting of aryl, heteroaryl, -OH, -SH, $C_1$-$C_4$-alkyl, -O-($C_1$-$C_4$-alkyl), -S($C_1$-$C_4$-alkyl), $-CF_3$, Cl, Br, F, I, $-NO_2$, $-CO_2H$, $CO_2$-($C_1$-$C_4$-alkyl), $-NH_2$, -NH($C_1$-$C_4$-alkyl), -N($C_1$-$C_4$-alkyl$)_2$, $-PO_3H_2$, -PO(OH)(O-$C_1$-$C_4$-alkyl), $-PO(OR^4)R^9$, morpholinyl, $-SO_xR^4$, piperazinyl-4-$COR^{22}$, or $C_1$-$C_4$ alkylpiperazinyl,
(f) perfluoro-$C_1$-$C_4$-alkyl, or
(g) $CH_2CH_2$($C_2$-$C_4$-alkenyl);

X is

(a) a carbon-carbon single bond,
(b) -CO-,
(c) -O-,
(d) -S-,
(e)

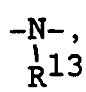

(f)

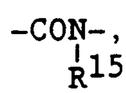

(g)

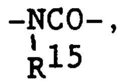

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,
(p) $-CH=CH-$,
(q) $-CF=CF-$,
(r) $-CH=CF-$,
(s) $-CF=CH-$,
(t) $-CH_2CH_2-$,
(u) $-CF_2CF_2-$,
(v)

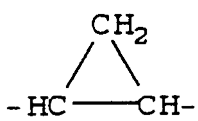

or

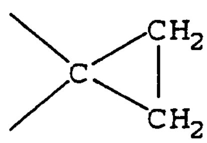

(w)

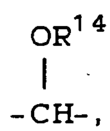

(x)

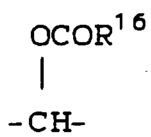

(y)

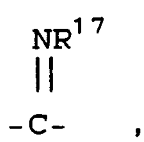

or
(z)

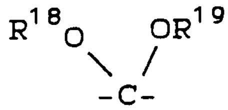 ;

r is 1 or 2,

x is 0 to 2,

and the pharmaceutically acceptable salts thereof.

The terms "alkyl," "alkenyl," "alkynyl," and the like include both the straight chain and branched chain species of these generic terms wherein the number of carbon atoms in the species permit. Unless otherwise noted, the specific names for these generic terms shall mean the straight chain species. For example, the term "butyl" shall means the normal butyl substituent, n-butyl.

One embodiment of this invention is represented by the compounds of the formula (I) wherein:

$R^1$ is

(a) $-CO_2R^4$,

(b)

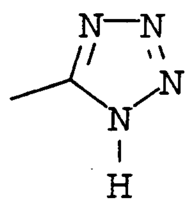 ,

(c) $-NH-SO_2R^{22}$;

(d) $-SO_2NH$-heteroaryl,

(e) $-CH_2SO_2NH$-heteroaryl,

(f) $-SO_2NH-CO-R^{22}$,

(g) $-CH_2SO_2NH-CO-R^{22}$,

(h) $-CONH-SO_2R^{22}$,

(i) $-CH_2CONH-SO_2R^{22}$,

(j) $-NHSO_2NHCO-R^{22}$,

(k) $-NHCONHSO_2-R^{22}$, or

(l) $-SO_2NHCN$;

$R^{2a}$ is H;

$R^{2b}$ is H, F, Cl, $CF_3$, $NO_2$, $C_1$-$C_6$-alkyl, $C_2$-$C_6$-alkenyl, $C_2$-$C_6$-alkynyl, or aryl;

$R^{3a}$ is H;

$R^{3b}$ is H, F, Cl, $CF_3$, $NO_2$, $C_1$-$C_4$-alkyl, $C_2$-$C_4$-alkenyl, $C_2$-$C_4$-alkynyl, $C_5$-$C_6$-cycloalkyl, $-COOCH_3$, $-COOC_2H_5$, $-SO_2-CH_3$, $NH_2$, $-N(C_1-C_4\text{-alkyl})_2$ or $-NH-SO_2CH_3$;

E is a single bond, -O- or -S-;

$R^6$ is

(a) $C_1$-$C_5$ alkyl or a substituted $C_1$-$C_5$ alkyl with a substituent selected from the group consisting of $C_3$-$C_5$-cycloalkyl, Cl, $CF_3$, $CCl_3$, $-O-CH_3$, $-OC_2H_5$, $-S-CH_3$, $-S-C_2H_5$, phenyl, or F;

(b) $C_2$-$C_5$-alkenyl or $C_2$-$C_5$-alkynyl; or,

(c) $C_3$-$C_5$-cycloalkyl;

$R^{7a}$, $R^{7b}$, $R^{8a}$ and $R^{8b}$ are independently

(a) H,

(b) $C_1$-$C_8$-alkyl or a substituted $C_1$-$C_8$-alkyl with a COOR, $OCOR^{4a}$, OH, aryl, $-(CH_2)_{1-4}R^4$, $-CO-R^4$, -CO-heteroaryl,

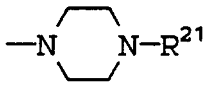, 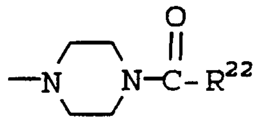,

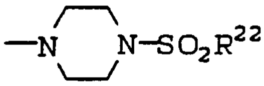 or 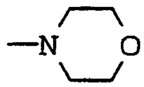

substituent,

(c) $OR^{22a}$,

(d) $-NO_2$,

(e)

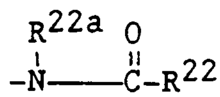,

(f) $-CONR^4R^{22}$,

(g)

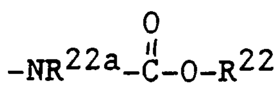,

(h) $-NR^4R^{22}$,

(i) Cl, F, or Br,

(j) $-CF_3$,

(k) $-CO_2R^4$,

(l) -CO-aryl,

(m) $-S(O)_x-R^{22}$,

(n) $-SO_2-NR^4R^9$,

(o) $-N(R^{22a})SO_2R^{22}$,

(p) aryl,

(q) heteroaryl,

(r) $-N(R^{22a})CONR^4R^{22}$,

(s) $-N(R^{22a})SO_2N(R^4)R^{22}$,

(t) $-N(R^{22a})SO_2OR^{22}$,

(u)

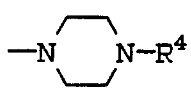,

(v)

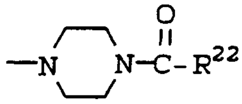,

(w)

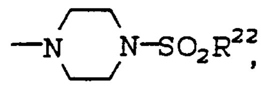

or

(x)

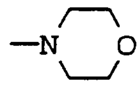 ;

$R^{21}$ is H, F, or Cl;

X is a single bond;

r is one.

A class of this embodiment is represented by the compound of the formula (I)

$R^1$ is

(a) $-CO_2R^4$,

(b)

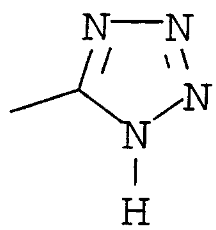 ,

(c) $-NH-SO_2-R^{22}$,

(d) $-SO_2NH$-heteroaryl,

(e) $-SO_2NH-CO-R^{22}$,

(f) $-CONH-SO_2R^{22}$, or

(e) $-SO_2NHCN$;

E is a single bond;

$R^{2b}$ and $R^{3b}$ independently are H, $-C_1$-$C_4$-alkyl, $-C_2$-$C_4$-alkenyl, $-C_2$-$C_4$-alkynyl, -Cl, -F, $NO_2$, or $CF_3$;

$R^6$ is $-C_1$-$C_4$-alkyl, -cyclopropyl, $-CH_2CH_2CH_2CF_3$, $CH_2CH_2CF_3$, $-C_2$-$C_5$-alkenyl, $-CH_2OCH_3$ or -cyclopropyl;

$R^{7a}$, $R^{7b}$, $R^{8a}$ and $R^{8b}$ are each independently H, $-C_1$-$C_4$-alkyl, $-NO_2$, $-NR^4R^{22}$, $-OCH_3$, $-N(R^{22a})$-$COOR^{22}$, -Cl, $-CH_2COOR^{4a}$, $-S(O)_x-R^{22}$, alkyl, $N(R^{22a})CON(R^4)R^{22}$, $CH_2OCO$-($C_1$-$C_4$-alkyl), $N(R^{22a})COR^{22}$, -F, $-CH_2Ph$, $-CONR^4R^{22}$, $CO_2R^4$, aryl, heteroaryl, $-C_1$-$C_4$-alkyl-heteroaryl as defined hereinabove,

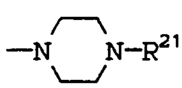 , 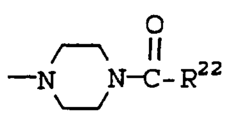 , 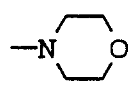 ,

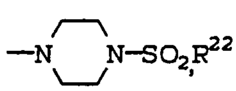 or 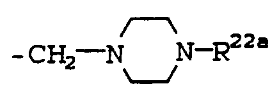

Illustrating this class are the compounds of the following structural formula (II)

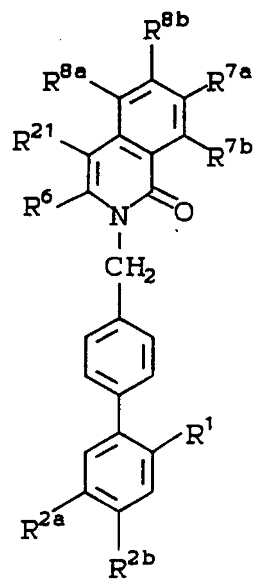

(II)

wherein:

$R^1$ is

(a) $-CO_2R^4$,

(b)

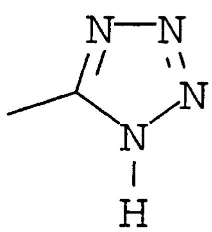

,

(c) $-SO_2NHCOR^{22}$,

(d) $-CONHSO_2R^{22}$,

(e) $-NHSO_2R^{22}$, or

(f) $-SO_2NHCN$;

$R^{2a}$ and $R^{2b}$ are independently hydrogen or $C_1$-$C_4$ alkyl;

$R^6$ is

(a) $-C_1$-$C_4$ alkyl,

(b) $-CH_2CH_2CH_2CF_3$,

(c) $-CH_2CH_2CF_3$,

(d) $-CH_2OCH_3$, or

(e) cyclopropyl;

$R^{7a}$ is

(a) hydrogen,

(b) $C_1$-$C_4$ alkyl,

(c) $-NR^4R^{22}$,

(d) $-NR^{22a}CONR^4R^{22}$,

(e) $-NR^{22a}COR^{22}$, or

(f) $-NR^{22a}CO_2R^{22}$;

(g) aryl as defined hereinabove,

(h) heteroaryl as defined hereinabove,

(i)

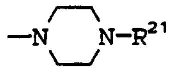 ,

(j)

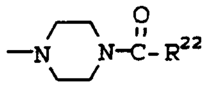 ,

(k)

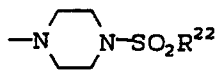 ,

(l)

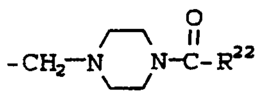 ,

(m)

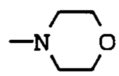 ,

or
(n)

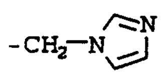 ;

$R^{7b}$ is
(a) hydrogen
(b) $C_1$-$C_4$ alkyl,
(c) F, or
(d) $CO_2R^4$;

$R^{8a}$ is
(a) hydrogen,
(b) $C_1$-$C_4$ alkyl,
(c) F;

$R^{8b}$ is
(a) hydrogen,
(b) $C_1$-$C_4$ alkyl,
(c) -$CO_2R^4$;

$R^{21}$ is hydrogen or F.

Exemplifying this class are the following compounds having the structure III:

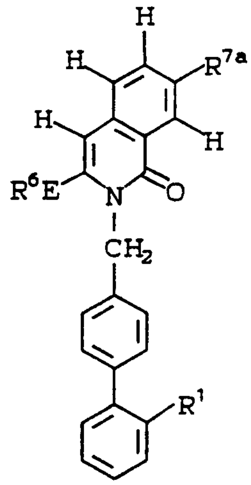

(III)

| $R^1$ | $ER^6$ | $R^{7a}$ |
|---|---|---|
| TET | Et | $N(Bn)CO_2Bn$ |
| TET | Et | $N(Bn)CO_2tBu$ |
| TET | Et | N(Bn)COPh |
| TET | Et | N(Bu)COPhpF |
| TET | Et | N(Bn-4-F)COPh |
| TET | Et | $N(Pn)SO_2Pr$ |
| TET | Et | $N(Pn)SO_2Bu$ |
| TET | Et | N(Bn)CONHPr |
| TET | c-Pr | N(Bz)Bn |
| TET | c-Pr | N(Bz)Pn |
| $SO_2NHBz$ | Et | N(Bz)Bn |
| TET | Me | N(Bz)Bn |
| TET | Me | N(Bn-2-Cl)Bz |
| TET | iPr | N(Bz)Bn |
| TET | Et | $N(SO_2Pr)Bn$ |
| TET | Et | N(CO-4-Pyr)Bn |
| TET | Et | N(Bn)CO(Ph-2-Cl) |
| TET | Et | N(Bn-2-Cl)CO(Ph-2-Cl) |
| TET | Et | N(Bn-2-Cl)COPh |
| TET | Et | N(Pn)COPh-4-F |
| TET | H | N(Bn)Bz |
| TET | H | N(Bn-2-Cl)Bz |
| TET | iPr | N(Bn)COcPr |
| TET | iPr | N(Bn-2-Cl)COcPr |

| | | |
|---|---|---|
| TET | iPr | N(Bn-2-Cl)Bz |
| TET | Et | N(iBu)Bz |
| TET | Et | N(i-pentenyl)Bz |
| TET | Et | N(Bn-3-Et)Bz |
| TET | Et | N(Bn-4-Cl)Bz |
| TET | Et | N(Bn-2-F)Bz |
| TET | Et-2-(Et) | N(Bn)Bz |
| TET | Me | N(Bn-2-Cl)CO-4-Pyr |
| TET | Me | N(i-pentenyl)CO-4-Pyr |
| TET | Et | N(Bn-4-I)Bz |
| TET | Et | N(Bn)CO(Ph-4-I) |
| TET | iPr | N(Bn-4-I)Bz |
| TET | Et | N(Bn-2-I)Bz |
| TET | Et | N(Bn)CO-2-thienyl |
| TET | Pr | N(Bn)CO-2-thienyl |
| TET | Et | N(Bn)CO-2-furoyl |
| $SO_2NHBz$ | Bu | N(Bn)COOiBu |
| $SO_2NHBz$ | Pr | N(Pn)Bz |
| $SO_2NHCOcPr$ | Pr | N(Pr)COMe |
| $SO_2NHCOcPr$ | Pr | N(Pn)COcPr |
| $SO_2NHCOcPr$ | Pr | N(Pn)COMe |
| $SO_2NHCOPh$ | Pr | N(Pr)COcPr |
| $SO_2NHCOcPr$ | Pr | N(Me)COMe |
| $SO_2NHBz$ | Pr | $N(Pn)CO_2Bn$ |
| TET | Pr | $N(Pn)CO(Ph-4-CF_3)$ |
| TET | c-Pr | N(Bz)Bn |

| | | |
|---|---|---|
| TET | Pr | N(Bu)CO-4-Pyr |
| TET | Pr | N(Pn)CO-3-Pyr |
| TET | Pr | N(Pn)CO(Ph-4-SMe) |
| TET | Pr | N(Pr)Bz |
| TET | Pr | N(Pn)CO(Ph-4-$SOCH_3$) |
| TET | Pr | N(Pn)CO(Ph-4-$SO_2$Me) |
| TET | Pr | N(Pr)COPhpF |
| TET | Pr | N(Bz)Bz |
| TET | Pr | N(iBu)Bz |
| TET | Pr | N($(CH_2)_3$COOEt)Bz |
| TET | Pr | N($(CH_2)_3$COOH)Bz |
| TET | Pr | N($CH_2$COOEt)Bz |
| TET | Pr | N($CH_2$-4-pyr)COOPr |
| TET | Pr | N(Pn)CO-2-thienyl |
| TET | Pr | N(Me)CO-2-thienyl |
| TET | Pr | N(Me)$CO_2(CH_2)_2$OMe |
| TET | Pr | N($CH_2CH_2$OMe)CO-4-Pyr |
| TET | Pr | N($CH_2$-3-Pyr)COOPr |
| TET | Pr | N($CH_2$-2-Pyr)COOPr |
| TET | Pr | N($CH_2$-4-Pyr)$CO_2(CH_2)$2OMe |
| TET | Pr | N($CH_2$COOH)Bz |
| TET | Pr | N(4-F-Bn)CON(Me)iPr |
| TET | Pr | N($CH_2$-2-Pyr)CON(Me)iPr |
| TET | $CH_2$OMe | N(Me)$CO_2$iBu |
| TET | $CH_2$OMe | N($CH_2$-2-Pyr)$CO_2$Pr |

| | | |
|---|---|---|
| TET | $CH_2OMe$ | N(Pn)CO-4-ClPh |
| TET | Pr | N(Et)CO-2-thienyl |
| TET | Pr | N(Et)CO-2-furoyl |
| TET | Pr | N(Bu)CO(Ph-2-OMe) |
| TET | Pr | N(Pr)CO(Ph-2-OMe) |
| TET | Pr | N(Pn)CO(Ph-4-OBn) |
| TET | Pr | N(Pr)CO(Ph-4-OBn) |
| TET | Pr | N(Pn)CO(Ph-4-OH) |
| TET | Pr | N(Bu)CO(Ph-4-OH) |
| TET | Pr | $N(CH_2$-2-Pyr)$CO_2Et$ |
| TET | Pr | N(Bn-4-$NO_2$)$CO_2Pr$ |
| TET | Pr | N(Bn-4-$NH_2$)$CO_2Pr$ |
| TET | Pr | N(Bn)$COCH_2$-N1-Imidazole |
| TET | Pr | N(Bn)$COCH_2$PIPBoc |
| TET | Pr | N(Bn)CO-3-Pyr |
| $SO_2NHBz$ | Pr | NHPn |
| $SO_2NHBz$ | Bu | 4-Me-Ph |
| $SO_2NHCOcPr$ | Bu | 4-Me-Ph |
| $SO_2NHBz$ | Bu | 2-Pyr |
| $SO_2NHBz$ | Pr | 4-Me-Ph |
| $SO_2NHBz$ | Pr | 2-Pyr |
| $SO_2NHCN$ | Pr | N(Pn)Bz |
| TET | Pr | $NHCOCH_2NMe_2$ |
| TET | Pr | $NH_2$ |
| TET | $CH_2OMe$ | $NH_2$ |
| TET | Pr | $NMe_2$ |
| TET | Pr | Ph-4-Me |

| | | |
|---|---|---|
| TET | Pr | Ph-2-($CO_2$tBu) |
| TET | Pr | $NHCH_2$PhMe |
| TET | Pr | 2-pyridyl |
| TET | Pr | 2-furoyl |
| TET | Pr | Ph-4-Cl |
| TET | Pr | Ph-2-COOH |
| TET | Pr | NHPr |
| TET | Pr | Ph |
| TET | Pr | Ph-4-OMe |
| TET | Pr | Ph-2,5-$(OMe)_2$ |
| TET | Pr | 3-Pyr |
| TET | Pr | 4-Pyr |
| TET | Bu | 4-Cl-Ph |
| TET | Bu | 2-Pyr |
| $SO_2$NHCOcPr | Pr | Me |
| $SO_2$NHBz | Pr | Me |
| $SO_2$NHCOPh | Pr | NHBz |
| $SO_2$NHCOPh | Pr | NHBz |
| $SO_2$NHCN | Pr | Me |
| $SO_2$NHCOcPr | Pr | NHBz |
| $SO_2$NHAc | Pr | NHBz |
| $SO_2$NHAc | Pr | NHBz |
| $SO_2$NHCO-2-furoyl | Pr | Me |
| $SO_2$NHCOcPr | Et | NHBz |
| $SO_2$NHBz | Et | NHBz |
| $SO_2$NHAc | Pr | NHAc |
| $SO_2$NHCOcPr | Pr | NHCOcPr |
| $SO_2$NHBz | Pr | NHCONHiPr |

| | | |
|---|---|---|
| $SO_2NHBz$ | Pr | NHCO-2-furyl |
| $SO_2NHAc$ | Pr | NHCO-2-furyl |
| $SO_2NHCO$-2-furoyl | Pr | NHCO-2-furoyl |
| $SO_2NHBz$ | Pr | NHCON(Me)iPr |
| $SO_2NHCOBu$ | Pr | NHCO-2-furoyl |
| $SO_2NHBz$ | Bu | NHCONHiPr |
| $SO_2NH$(CO-2-furyl-3-Me) | Pr | NHCO-2-furyl |
| $SO_2NHAc$ | Pr | NHCONHiPr |
| $SO_2NHCOBu$ | Pr | $NH_2$ |
| $SO_2NH$(CO-2-furyl-3-Me) | Bu | NHCONHEt |
| $SO_2NHCOBu$ | Pr | NHCONHMe |
| $SO_2NHCO(CH_2)_2OEt$ | Pr | NHCONHEt |
| $SO_2NHCOPn$ | Pr | NHCONHEt |
| TET | Et | Me |
| TET | Et | $NHSO_2Pr$ |
| TET | Et | NHBz |
| TET | Et | NH(CO-Ph-2-Cl) |
| TET | Et | NHCOBz |
| TET | Pr | $CH_2$-N-imidazole |
| TET | Pr | $CH_2PIPBOC$ |
| TET | Pr | $CH_2PIPCOcPr$ |
| TET | Pr | $N(CH_2$-2-Pyr)CON(Me)iPr |
| $SO_2NHCOcPr$ | Bu | morpholino |
| $SO_2NHCOcPr$ | Bu | PIPCOcPr |
| $SO_2NHCOcPr$ | Pr | PIP-2-Pyr |
| Ph-4-$SO_2NHCOcPr$ | Bu | PIP-2-Pyr |
| $SO_2NHCOcPr$ | Pr | PIPCOcPr |
| TET | Pr | morpholino |
| TET | Bu | PIPCOMe |
| $SO_2NHCOcPr$ | Bu | PIPCOMe |
| TET | Pr | PIPCOMe |
| $SO_2NHCOcPr$ | Pr | morpholino |
| TET | Bu | PIPCOcPr |

| | | |
|---|---|---|
| $SO_2NHCOcPr$ | Bu | PIP-2-Pyr |
| $SO_2NHCOcPr$ | Pr | PIPCOcPr |
| $SO_2NHBz$ | Pn | PIPCOcPr |
| $SO_2NHBz$ | Bu | PIPCOcPr |
| TET | Pn | PIPCOcPr |
| TET | Bu | $PIPSO_2iPr$ |
| $SO_2NHCN$ | Bu | PIPCOcPr |
| $SO_2NHCONHBu$ | Bu | PIPCOcPr |
| $SO_2NHCO(CH_2)_5NBoc$ | Bu | PIPCOcPr |
| $SO_2NHCO(CH_2)_5NH_2$ | Bu | PiPCOcPr |
| $SO_2NHCO(CH_2)_3iPr$ | Bu | PIPCOcPr |
| $SO_2NHCO(CH_2)_3cPr$ | Bu | PiPCOcPr |
| $SO_2NHCO(CH_2)_3CH_3$ | Bu | $NHCON(CH_3)iPr$ |
| $SO_2NHCOPn$ | Pr | NHCONHEt |
| $SO_2NHCOPn$ | Pr | NHCONHBu |
| $SO_2NHCO(CH_2)_3iPr$ | Pr | NHCONHMe |
| $SO_2NHCO(CH_2)_3NMe_2$ | Pr | NHCONHEt |
| $SO_2NHCO(CH_2)_3cPr$ | Pr | NHCONHEt |
| TET | Pr | N(Bn)CO-2-Pyr |
| TET | Pr | $N(CH_2\text{-}2\text{-}Pyr)Bz$ |
| $SO_2NHCOPn$ | Bu | PIPCOcPr |

| $R^1$ | $ER^6$ | $R^{7a}$ |
|---|---|---|
| $SO_2NHCO(CH_2)_4CH_3$ | Pr | NHCO 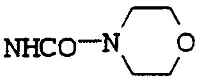 |
| $SO_2NHCO(CH_2)_4CH_3$ | Pr | NHCO 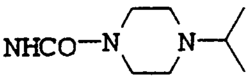 |
| $SO_2NHCO(CH_2)_4CH_3$ | Pr | NHCO 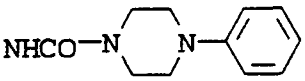 |

PIP = 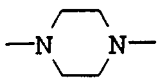 ; TET = 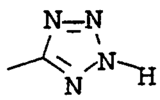

-CO(Ph-2-OMe) = 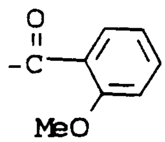

-Bn-4-I = 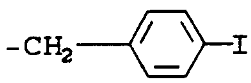

Pn = n-pentyl

Further exemplifying this class are the following compounds having the structure IV:

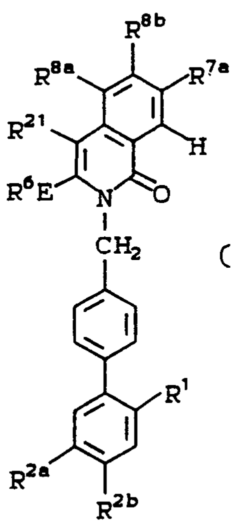

(IV)

| $R^1$ | $R^{2a}$ | $R^{2a}$ | $ER^6$ | $R^{21}$ | $R^{8a}$ | $R^{8b}$ | $R^{7a}$ |
|---|---|---|---|---|---|---|---|
| COOH | H | H | Bu | H | H | H | H |
| TET | H | H | Pr | H | H | H | H |
| TET | H | H | Pr | H | Me | H | H |
| TET | H | H | Pr | F | H | H | H |
| TET | H | H | Pr | H | H | H | N(n-Pn)Bz |
| TET | H | H | Pr | H | H | H | Me |
| TET | H | H | c-Pr | H | H | H | H |
| TET | H | H | Pr | H | H | H | $N(n\text{-}Bu)CO_2\text{-}n\text{-}Bu$ |
| TET | H | H | Pr | H | H | H | N(n-Bu)COPh-4-F |
| $SO_2NHCOPh\text{-}4\text{-}F$ | H | H | Et | H | H | i-Pr | H |
| $SO_2NHCOPh$ | H | H | Pr | H | H | H | N(Me)CONHiPr |
| $SO_2NHCOPh$ | H | H | Pr | H | F | H | N(Me)CON(Me)iPr |
| TET | H | H | Bu | H | H | COOH | H |
| TET | H | H | Et | H | H | H | $N(n\text{-}Pn)COPh\text{-}4\text{-}CF_3$ |
| $SO_2NHCOPh$ | H | H | Pr | H | H | H | N(n-Pn)COPh |
| $SO_2NHCOcPr$ | H | H | Pr | H | H | H | N(n-Pn)COPh |
| $SO_2NHCOCH_2NH_2$ | H | H | Pr | H | H | H | N(n-Pn)CO-4-Cl |
| $SO_2NHCOPn$ | Pr | H | Pr | H | H | H | NHCONHEt |
| $SO_2NHCOPn$ | Pr | H | Bu | H | H | H | NHCONHEt |
| $SO_2NHCOBu$ | Pr | H | Bu | H | H | H | NHCONHEt |
| $SO_2NHCO(CH_2)_3OMe$ | Pr | H | Pr | H | H | H | NHCONHEt |
| $SO_2NHCOPn$ | Pr | H | Bu | H | H | H | 2-Pyridyl |
| $SO_2NHCOPn$ | H | Pr | Pr | H | H | H | NHCONHEt |
| $SO_2NHCOPn$ | H | Pr | Bu | H | H | H | NHCONHEt |
| $SO_2NHCOBu$ | H | Pr | Bu | H | H | H | NHCONHEt |
| $SO_2NHCO(CH_2)_3OMe$ | H | Pr | Pr | H | H | H | NHCONHEt |
| $SO_2NHCOPn$ | H | Pr | Bu | H | H | H | 2-Pyridyl |
| TET | H | Pr | Pr | H | H | H | $N(CH_2\text{-}2\text{-}Pyr)Bz$ |
| TET | H | H | Pr | H | H | H | $N(CH_2\text{-}2\text{-}Pyr)CO\text{-}2\text{-}Pyr$ |
| TET | H | H | Pr | H | H | H | N(Bn)CO-2-Pyr |
| TET | H | H | Pr | H | H | H | $N(Bn)CO_2Et$ |
| TET | H | H | Pr | H | H | H | N(Pn)CO-4-Pyr |
| TET | H | H | Pr | H | H | H | $NHCON(CH_3)iPr$ |

The compounds of Formula (I) can be synthesized using the reactions and techniques described below. The reaction are performed in a solvent appropriate to the reagents and materials employed and suitable for the transformation being effective. It is understood by those skilled in the art of organic synthesis that the functionality present on the heterocycle and other parts of the structure should be consistent with the chemical transformations proposed. Depending upon the reactions and techniques employed, this may involve changing the order of the synthetic steps, the use of required protecting groups followed by deprotection, and, depending upon the particular pyrimidinone fused heterocycle being formed, the use of different strategies may be employed regarding the cyclization steps and the particular starting materials utilized.

## ABBREVIATIONS USED IN REACTION SCHEMES

### Reagents:

| | |
|---|---|
| NBS | N-bromosuccinimide |
| AIBN | azo(bis)isobutyronitrile |
| DDQ | dichlorodicyanoquinone |
| $Ac_2O$ | acetic anhydride |
| TEA | triethylamine |
| DMAP | 4-dimethylaminopyridine |
| $PPh_3$ | triphenylphosphine |
| TFA | trifluoroacetic acid |
| TMS-Cl | trimethylsilyl chloride |
| Im | imidazole |
| AcSK | potassium thioacetate |
| p-TsOH | p-toluenesulfonic acid |

### Solvents:

| | |
|---|---|
| $Et_2O$ | diethyl ether |
| DMF | dimethylformamide |
| HOAc (AcOH) | acetic acid |
| EtOAc (EtAc) | ethyl acetate |
| Hex | hexane |
| THF | tetrahydrofuran |
| DMSO | dimethylsulfoxide |
| MeOH | methanol |
| iPrOH | isopropanol |
| DBU | 1,8-diazabicyclo-[5.4.0] undec-7-ene |
| $Me_3SnCl$ | trimethylstannyl chloride |

Others:

| | |
|---|---|
| rt | room temperature |
| TBDMS | t-butyldimethylsilyl |
| OTf | $OSO_2CF_3$ |
| OTs | $OSO_2$-(4-methyl)phenyl |
| OMs | $OSO_2CH_3$ |
| Ph | phenyl |
| FAB-MS (FABMS) | Fast atom bombardment mass spectroscopy |
| NOE | Nuclear Overhauser Effect |
| $SiO_2$ | silica gel |
| trityl | triphenylmethyl. |
| s.b. | single bond |

Compounds of Formula (I) may be prepared via intermediate 4 by alkylation of the anion of 1(2H) isoquinolinone 2 with alkylating agent 3 (Scheme 1). Alternatively, the isoquinolinone may be converted to a silyl imine and then regioselectively alkylated on nitrogen by treatment with the alkylating agent in the presence of fluoride ion. Deprotection of the $R^1$ protecting group of intermediate product 4 will give rise to compounds of Formula I. Alternatively, if $R^1$ is nitrile and a tetrazole is desired, trimethyltin azide will convert the intermediate 4 to 1 where $R^1$ is tetrazole.

SCHEME 1

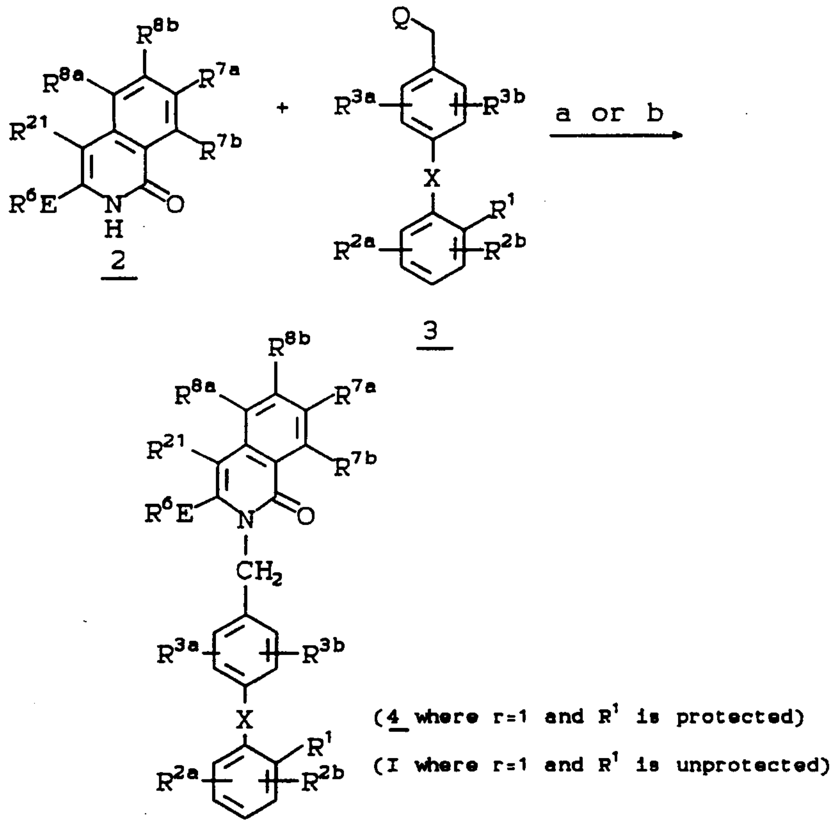


2

3

(4 where r=1 and $R^1$ is protected)

(I where r=1 and $R^1$ is unprotected)

Q = halogen, -O-tosyl or -O-mesyl

a. NaH, DMF

b. (i) $(CH_3)_3Si)_2NH$ or $(CH_3)_3SiCl/Et_3N$ (ii) 3, $F^-$

Isoquinolinones 2 and 2,3-disubstituted isoquinolinones 4 may be prepared from isocoumarins 6 - (Scheme 2). There are many synthetic approaches available to isocoumarins. [Compendium of Heterocyclic Chemistry] Treatment of an isocoumarin with ammonia will give rise to the isoquinolinones 2. Treatment of the isocoumarin with an alkyl amine 7 will give rise to the 2-substituted isoquinolinone 4. Both 2 and 4 may then be transformed into compounds of Formula I as shown in Scheme 1.

SCHEME 2

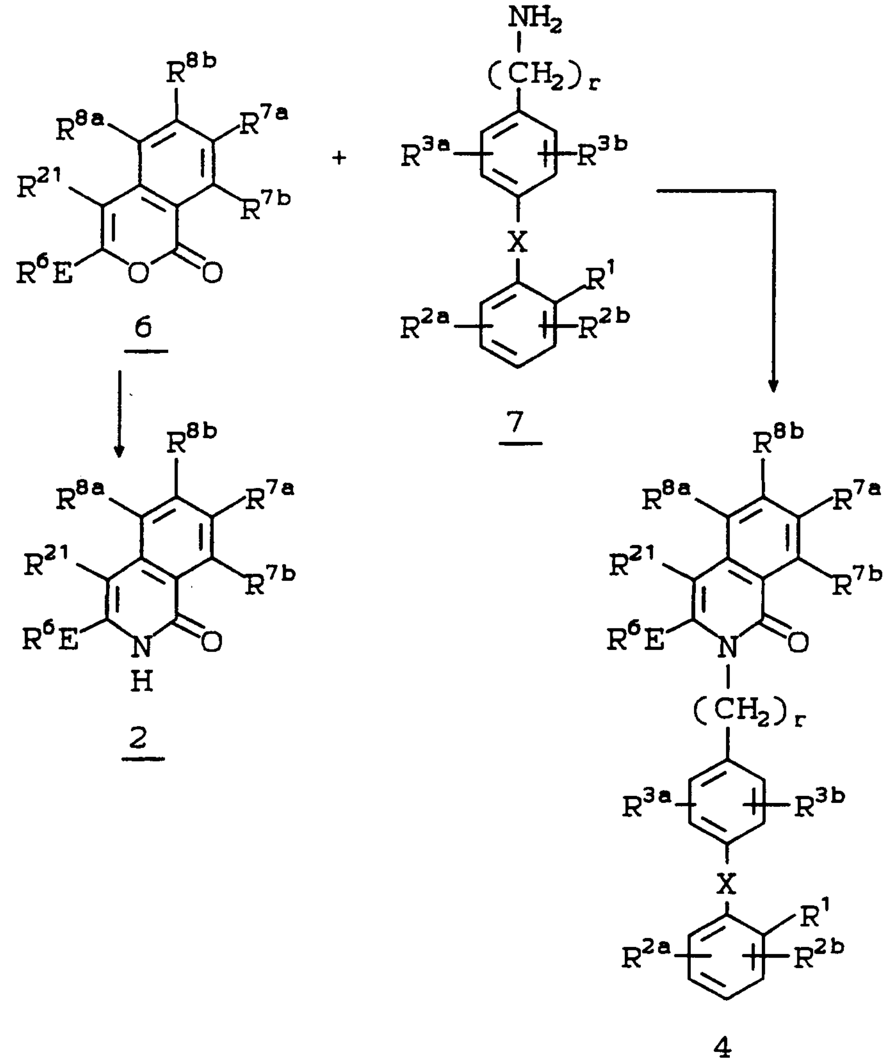

6 7 2 4

One of the most useful methods of preparing 1(2H)-isoquinolinones is from homophthalic anhydrides 8 - (Scheme 3). [R.B. Tirodkar, R.N. Usgaonkar. Indian J. Chemistry, 1060, 1972] Acylation of 8 under basic conditions with anhydrides gives rise to intermediate 4-acylisochroman-1,3-diones 9. In a similar fashion the diacid 10 may also be converted to 9. Treatment of 9 with ammonia will give rise to 1(2H)-isoquinolinones 2. Alternatively, acid treatment results in decarboxylation and formation of an isocoumarin 6. Both 2 and 6 may in turn be converted to compounds of Formula I by following Schemes 1 and 2 respectively.

SCHEME 3

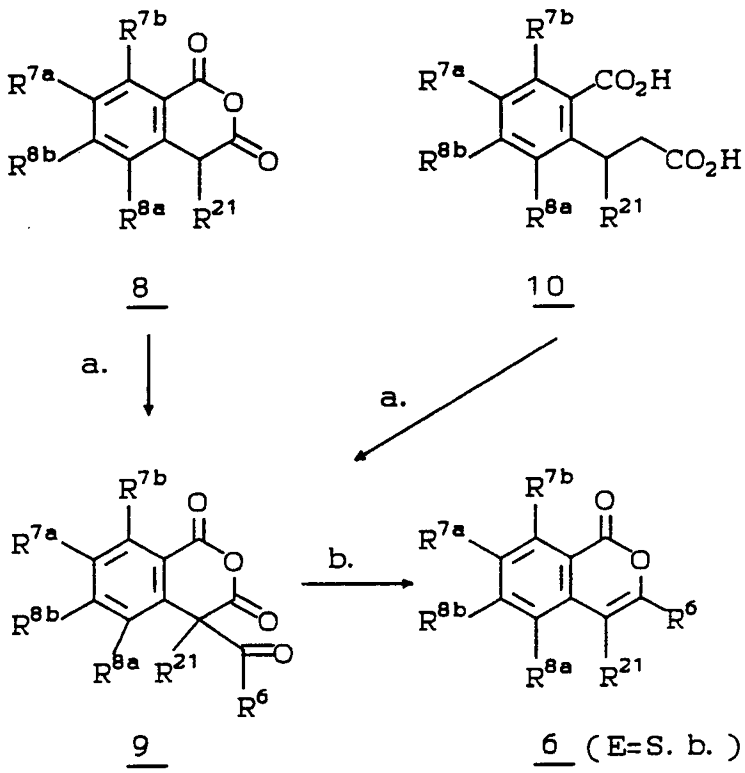


8 10 9 6 (E=S. b.)

a. $(R^6CO)_2O$, py
b. $H_2SO_4$, 95 ° C.

There are several other direct routes to 1(2H)-isoquinolinones 2 shown in Scheme 4. Treatment of 11 with an alkyl metal enolate and irradiation will give rise directly to a 1(2H)-isoquinolinone 2. [R. Beugelmans, M. Bois-Coussy. Synthesis. 729, 1981] Similarly, o-iodo benzamides 12 may be alkylated with copper acetylides in pyridine to give intermediate acetylide 13. These may be cyclized to 2 under palladium catalysis in the presence of sodium hydride in THF to give 2. [A. Nagarajam, T.R. Balasubramanian. Ind. J. Chem. 67, 1989] 2-Substituted indanones 14 may be rearranged to intermediate 2-alkyl-N-hydroxy-1(2H)-isoquinolinones 15 by treatment with an alkyl nitrite in the presence of acid. Reduction of 15 with, for example, iodine and red phosphorous will give 2. [E.J. Moriconi, F.J. Creegan. J. Org. Chem. 31, 2090, 1966] 2-N-Methyl benzamides 16 may be converted to the intermediate dilithio species with n-butyl lithium. Addition of an alkyl nitrile followed by acid gives rise to 2. [G.S. Poindexter. J. Org. Chem. 3787, 1982] The 1-(2H)-isoquinolinones may be converted to compounds of formula I by following the chemistry described in Scheme 1.

SCHEME 4

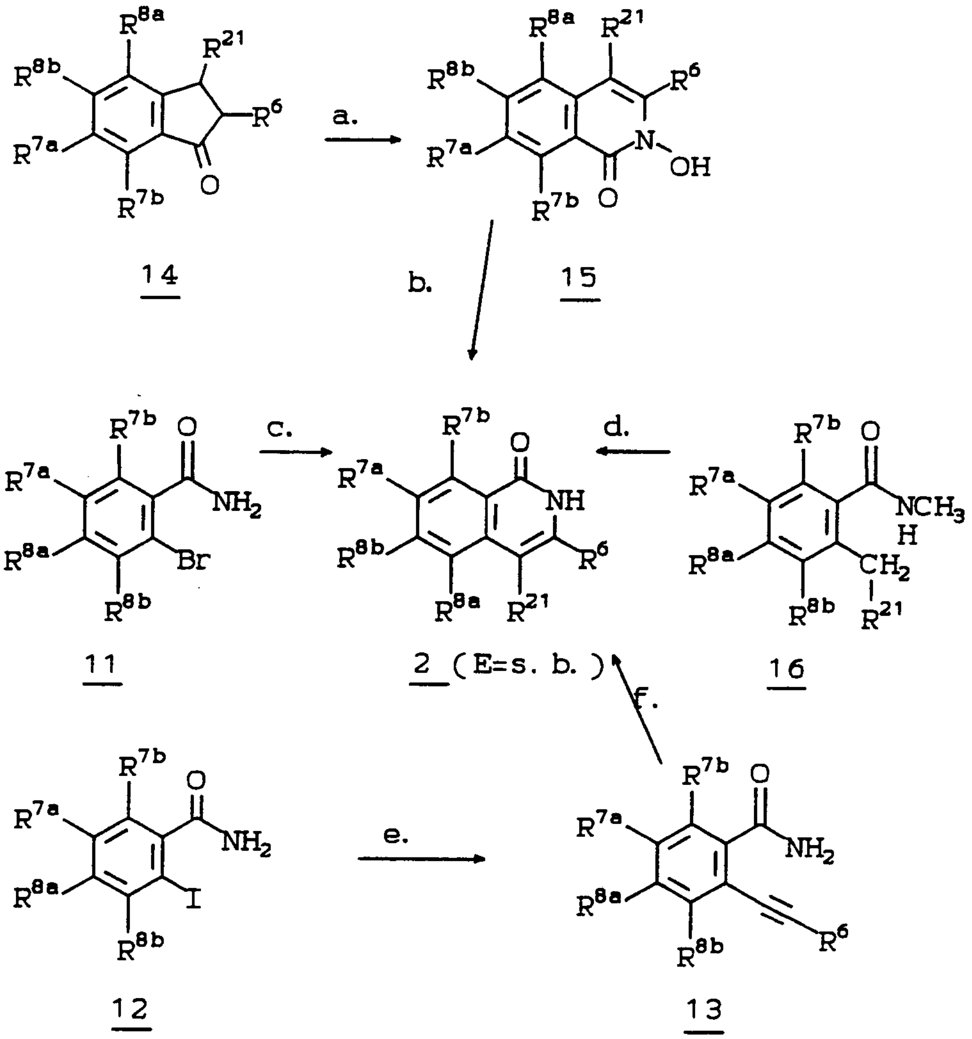


a. (i) $nBuNO_2$, $H_2SO_4$ (ii) MeOH, Δ
b. $I_2$, P
c.

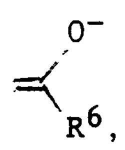

hv
d. (i) BuLi, (ii) $R^6CN$, (iii) $H^+$
e. Cu-C≡C -$R^6$, pyridine
f. $PdCl_2$, $CH_3CN$, NaH, THF.

Two alternative routes directly to 2,3-disubstituted isoquinolinones 5 may be available as shown in Scheme 5. Irradiation of N-vinyl benzamides 18 in methanol followed by oxidation by treatment with iodine will give rise to 2,3-disubstituted isoquinolinones 5. [A. Couture, P. Grandclaudin. Synthesis, 576, 1985] Alternatively, 2-methyl benzamides 19 may be dilithiated with n-butyl lithium and treated with an amide 20 to give the 2,3-disubstituted isoquinolinone 5. Compound 5 may then be converted to (I) as shown in Scheme 2.

SCHEME 5

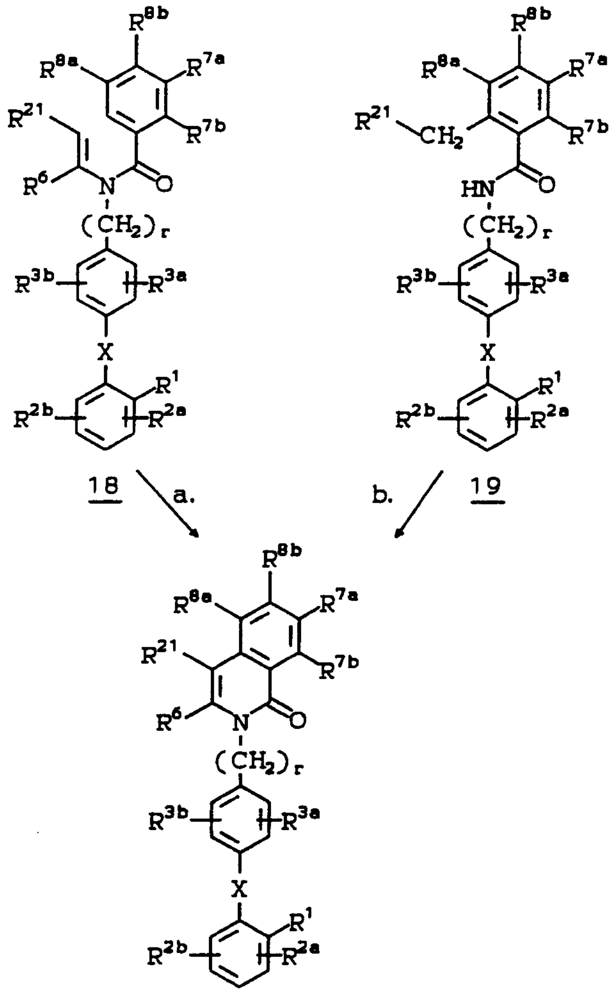


18 19

(5) (E=s. b.)

a. hv, argon, $CH_3OH$, $I_2$

b. (i) BuLi, (ii) $R^6CON(CH_3)_2$.

The benzyl halides (3) including the more preferred alkylating agents (21a and 21b, Scheme 6) can be prepared as described in European Patent Applications 253,310 and 291,969 and the references cited therein. However, a preferred method to prepare the biphenyl precursors 22a, 22b and 22b, using Ni(O) or Pd(O) catalyzed cross-coupling reaction [E. Negishi, T. Takahashi, and A. O. King, Org. Synthesis, 66, 67 (1987)], is outlined in Scheme 6. As shown in Scheme 6, treatment of 4-bromotoluene (23) with t-BuLi, followed by the addition of a solution of $ZnCl_2$, produces the organo-zinc compound (24). Compound (24) is then coupled with 25a or 25b in the presence of $Ni(PPh_3)_2Cl_2$ catalyst to produce the desired biphenyl compound 22a or 22b ($PPh_3$ = triphenylphosphine). Similarily, 1-iodo-2-nitro-benzene (25c) is coupled with organo-zinc compound 24 in the presence of $Pd(PPh_3)_4$ catalyst [prepared by treating $Cl_2Pd(PPh_3)_2$ with $(i\text{-}Bu)_2AlH$ (2 equiv.)] to give the biphenyl compound 22c. These precursors, 22a, 22b and 22c, are then transformed into halomethylbiphenyl derivatives 21a, 21b and 21c, respectively, according to procedures described in European Parent Applications 253,310 and 291,969.

SCHEME 6

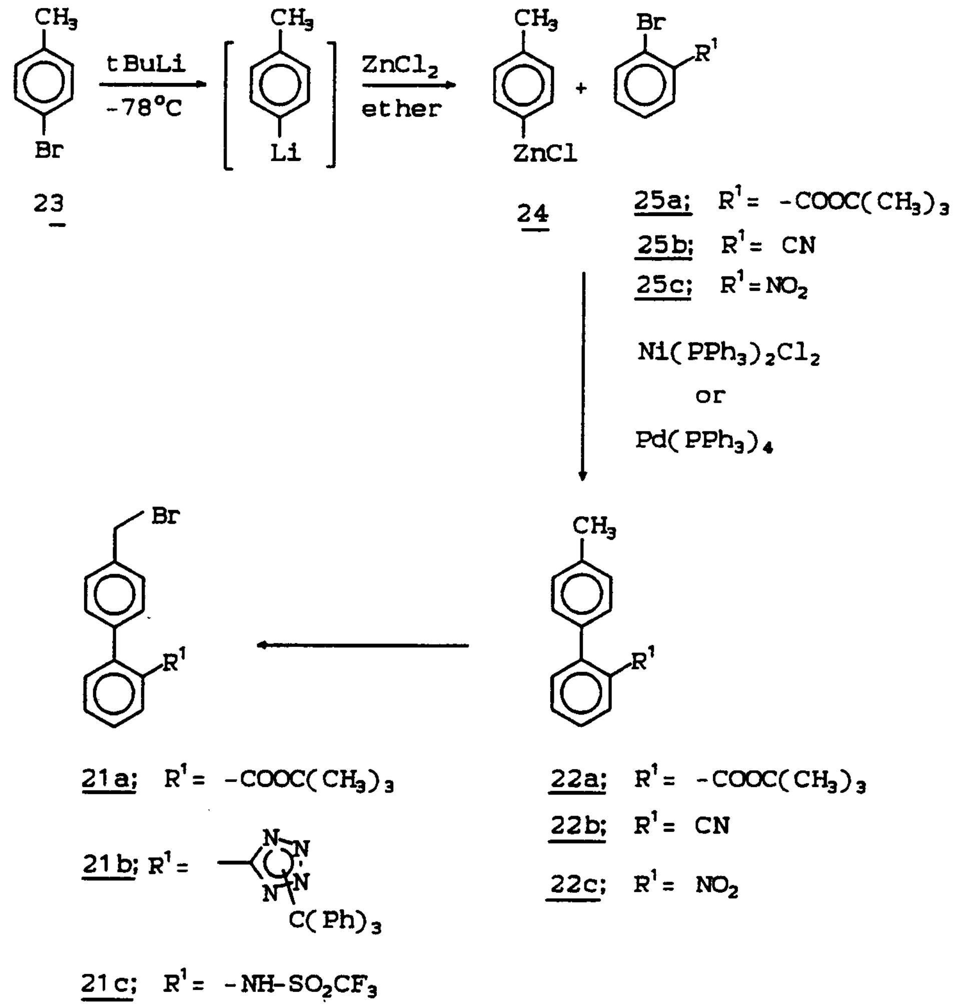


23

24

25a; $R^1$ = $-COOC(CH_3)_3$
25b; $R^1$ = CN
25c; $R^1$ = $NO_2$

21a; $R^1$ = $-COOC(CH_3)_3$
21b; $R^1$ =
21c; $R^1$ = $-NH-SO_2CF_3$

22a; $R^1$ = $-COOC(CH_3)_3$
22b; $R^1$ = CN
22c; $R^1$ = $NO_2$

When there is additional substitution on the second phenyl ring ($R^{2a}$, $R^{2b}$ = hydrogen) the preferred method to prepare the biphenyl precursors 22d and 22e, using the Pd(O) catalyzed cross-coupling reaction [J. K. Stille, Angew. Chem. Int. Ed. Engl., 25, 508 (1986)], is outlined in reaction Scheme 7. As shown in Scheme 7, p-tolyltrimethyltin (26) is coupled with 25d or 25e in refluxing toluene in the presence of 5 mole % of $Pd(PPh_3)_4$ to produce the desired biphenyl compounds 22d and 22e. Table I illustrates the synthetic utility of this protocol. Compounds 22d ($R^2$ = $NO_2$) and 22e ($R^2$ = $NO_2$) could be converted to their respective chlorides by catalytic hydrogenation, diazotization and treatment with copper (I) chloride. The biphenyl fluorides which could not be obtained by direct coupling to a fluoro arylbromide were prepared from 22d ($R^2$ = $NO_2$) and 22e ($R^2$ = $NO_2$) via reduction, formation of the diazonium tetrafluoroborate salt and thermal decomposition. These precursors 22d ($R^2$ = $NO_2$ or F or Cl) and 22e ($R^2$ = $NO_2$ or F or Cl) are then transformed into the halomethyl biphenyl derivatives 21d and 21e, respectively according to the procedures described in European Patent Applications 253,310 and 292,969.

## Biphenyl Synthesis Table I

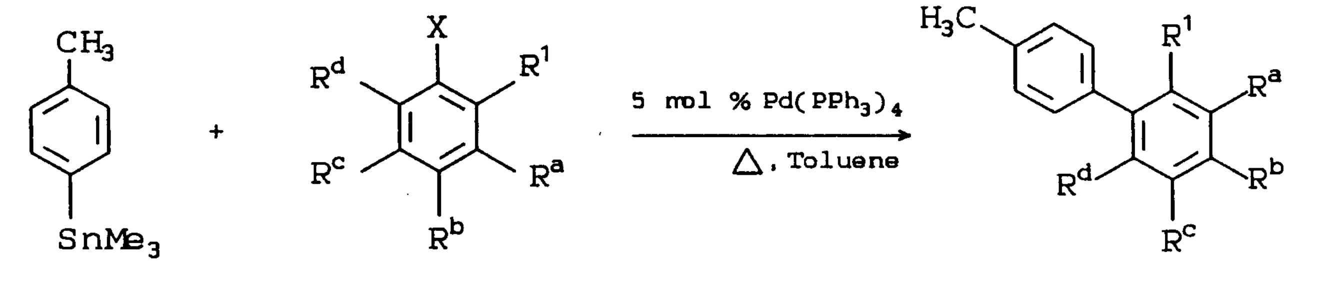


26 (25d, 25e) (22d, 22e)

| X | $R^1$ | $R^a$ | $R^b$ | $R^c$ | $R^d$ | Product ($R^a$) | Rf (solvent) | Yield |
|---|---|---|---|---|---|---|---|---|
| Br | $CO_2Me$ | $NO_2$ | H | H | H | 22d (3'-nitro) | 0.35(15:1 Hex/EtOAc) | 71% |
| Br | CN | H | $NO_2$ | H | H | 22e (4'-nitro) | 0.62(2x 6:1 Hex/EtOAc) | 74% |
| Br | $CO_2Me$ | H | F | H | H | 22d (4'-fluoro) | 0.43(15:1 Hex/EtOAc) | 83% |
| Cl | $CO_2Me$ | H | H | $NO_2$ | H | 22d (5'-nitro) | 0.22(15:1 Hex/EtOAc) | 70% |
| Br | $CO_2Me$ | H | H | H | $NO_2$ | 22d (6'-nitro) | 0.24(15:1 Hex/EtOAc) | 79% |
| Br | CN | H | F | H | H | 22e (4'-fluoro) | 0.44(15:1 Hex/EtOAc) | 64% |
| Cl | CN | H | H | F | H | 22e (5'-fluoro) | 0.40(15:1 Hex/EtOAc) | 62% |

REACTION SCHEME 7

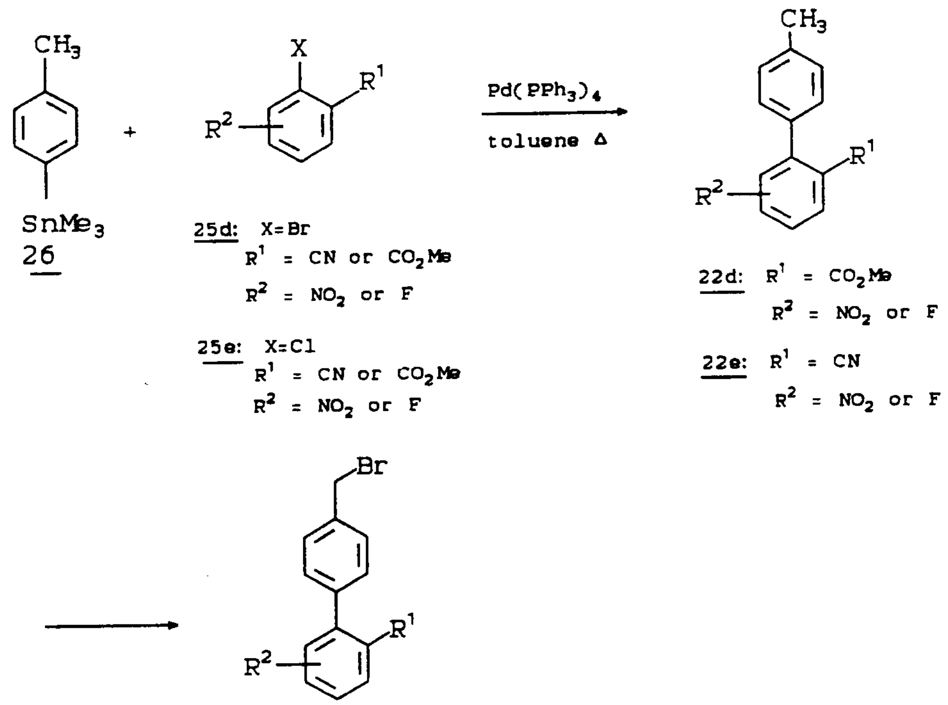


26

25d: X=Br
$R^1$ = CN or $CO_2Me$
$R^2$ = $NO_2$ or F

25e: X=Cl
$R^1$ = CN or $CO_2Me$
$R^2$ = $NO_2$ or F

22d: $R^1$ = $CO_2Me$
$R^2$ = $NO_2$ or F

22e: $R^1$ = CN
$R^2$ = $NO_2$ or F

21d: $R^1$ = $CO_2Me$
$R^2$ = $NO_2$ or F or Cl

21e: $R^1$ = $CN_4$-$CPh_3$
$R^2$ = $NO_2$ or F or Cl

Compounds of formula I where $R^1$ is $-CONHSO_2R^{22}$ (where $R^{22}$ = alkyl, aryl or heteroaryl) may be prepared from the corresponding carboxylic acid derivatives (27) as outlined in Scheme 8. The carboxylic acid (27), obtained as described in Scheme 2, can be converted into the corresponding acid chloride by treatment with refluxing thionyl chloride or preferably with oxalyl chloride and a catalytic amount of dimethylformamide at low temperature [A.W. Burgstahler, L.O. Weigel, and C.G. Shaefer-Synthesis, 767, (1976)]. The acid chloride then can be treated with the alkali metal salt of $R^{22}SO_2NH_2$ to form the desired acylsulfonamide (28). Alternatively, these acylsulfonamides may be prepared from the carboxylic acids using N,N-diphenylcarbamoyl anhydride intermediates [F.J. Brown et al, European Patent Application, EP 199543; K.L. Shepard and W. Halczenko- J. Het. Chem., 16, 321 (1979)]. Preferably the carboxylic acids can be converted into acyl-imidazole intermediates, which then can be treated with an appropriate aryl or alkylsulfonamide and diazabicycloundecane (DBU) to give the desired acylsulfonamide 28 [J.T. Drummond and G. Johnson, Tetrahedron. Lett., 28, 1653 (1988)].

SCHEME 8

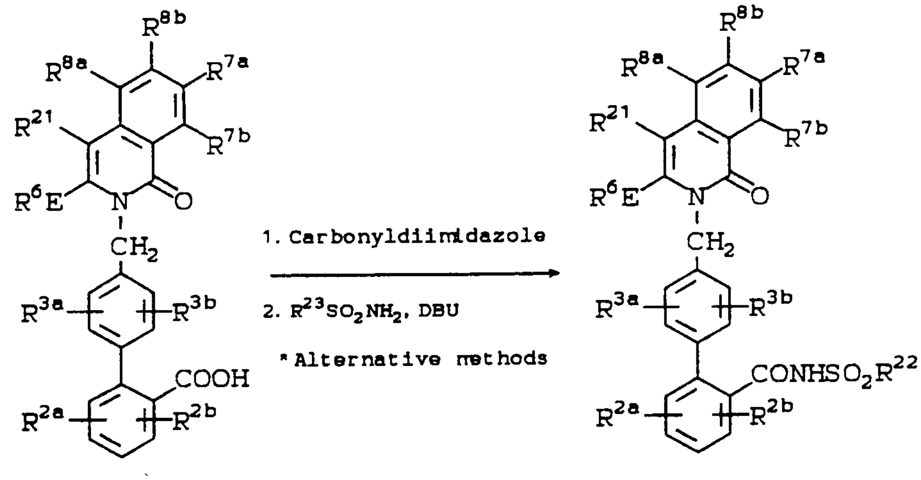


27 28

*Alternative Methods:

a)
(i) $SOCl_2$, reflux
(ii) $R^{22}SO_2NH^-M^+$ (where M is Na or Li)
b)
(i) $(COCl)_2$-DMF, -20°C
(ii) $R^{22}SO_2NH^-M^+$
c)
(i) N(N,N-Diphenylcarbamoyl)pyridinium chloride/aq. NaOH
(ii) $R^{22}SO_2NH^-M^+$.

Compounds of formula I where $R^1$ is $SO_2NHCOR^{22}$ or $SO_2NHCN$ may be prepared as outlined in Scheme 9. The nitro compound, for example 22c (prepared as described in Scheme 6), can be reduced to the corresponding amino compound and converted into aromatic diazoniun chloride salt, which then can be reacted with sulfur-dioxide in the presence of a copper (II) salt to form the corresponding arylsulfonyl chloride 29 [H. Meerwein, G. Dittmar, R. Gollner, K. Hafner, F. Mensch and O. Steifort, Chem. Ber., 90, 841 (1957); A.J. Prinsen and H. Cerfontain, Recueil, 84, 24 (1965); E.E. Gilbert, Synthesis, 3 (1969) and references cited therein]. The sulfonyl chloride can be reacted with ammonia in aqueous solution or in an inert organic solvent [F.H. Bergheim and W. Baker, J. Amer. Chem. Soc., 66, (1944), 1459], or with dry powdered ammonium carbonate, [E.H. Huntress and J.S. Autenrieth, J. Amer. Chem. Soc., 63 (1941), 3446; E.H. Huntress and F.H. Carten, J. Amer. Chem. Soc., 62, (1940), 511] to form the sulfonamide 30. The sulfonamide must then be protected preferably with the triphenylmethyl group by reaction with triphenylmethylchloride and triethylamine to give 31. The benzyl bromide 32 may be prepared from the sulfonamide 31 as outlined in Scheme 6, and then can be reacted with an alkali metal salt of an appropriate heterocyclic compound to form the key sulfonamide 33. The sulfonamide 33 may then be acylated under appropriate conditions to give 34. N-Cyanosulfonamides of structure 34a may be prepared by alkylation of the sodium salt of 33 with cyanogen bromide. The sulfonamide 33 may be also prepared from the aromatic sulfonyl chloride 36 by treatment with ammonia. Compound 36 may be prepared from the aryl amine 35 as outlined in Scheme 10.

SCHEME 9

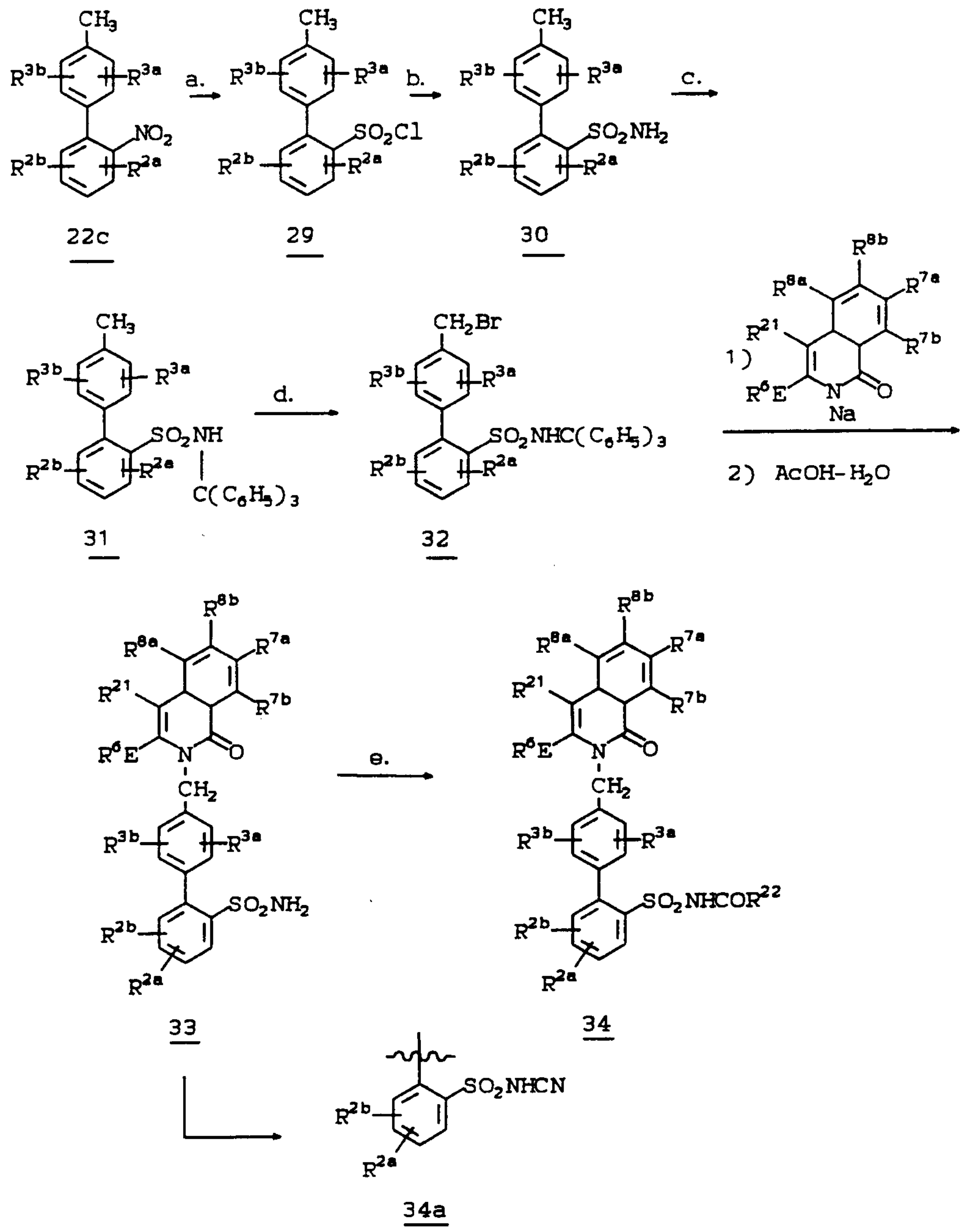


22c 29 30

31 32

33 34

34a

a.

(i) $H_2$/Pd-C,

(ii) $NaNO_2$-HCl,

(iii) $SO_2$, AcOH, $CuCl_2$

b. $NH_3$ or $(NH_4)_2CO_3$

c. $(C_6H_5)_3CCl$, $Et_3N$, $CH_2Cl_2$, 25° C

d. N-Bromosuccinimide

e. $R^{22}COCl$ or $R^{22}CO$-Im or other acylating agents.

The compounds bearing $R^1$ as $-SO_2NHR^y$ (where $R^y$ is heteroaryl or $R^9$) may be prepared by reacting the aromatic sulfonyl chloride 36 with appropriate heteroaryl amines as outlined in Scheme 10 to give 37. The sulfonyl chloride 36 may be prepared using similar chemistry to that outlined above. The sulfonyl chloride 36 may be the preferred intermediate for the synthesis of this class of compounds. The aromatic sulfonyl chlorides may also be prepared by reacting the sodium salt of aromatic sulfonic acids with $PCl_5$ or $POCl_3$ [C.M. Suter, The Organic Chemistry of Sulfur, John Wiley & Sons, 459, (1944)]. The aromatic

sulfonic acid precursors may be prepared by chlorosulfonation of the aromatic ring with chlorosulfonic acid [E.H. Huntress and F.H. Carten, J. Amer. Chem. Soc., 62, 511 (1940)].

SCHEME 10

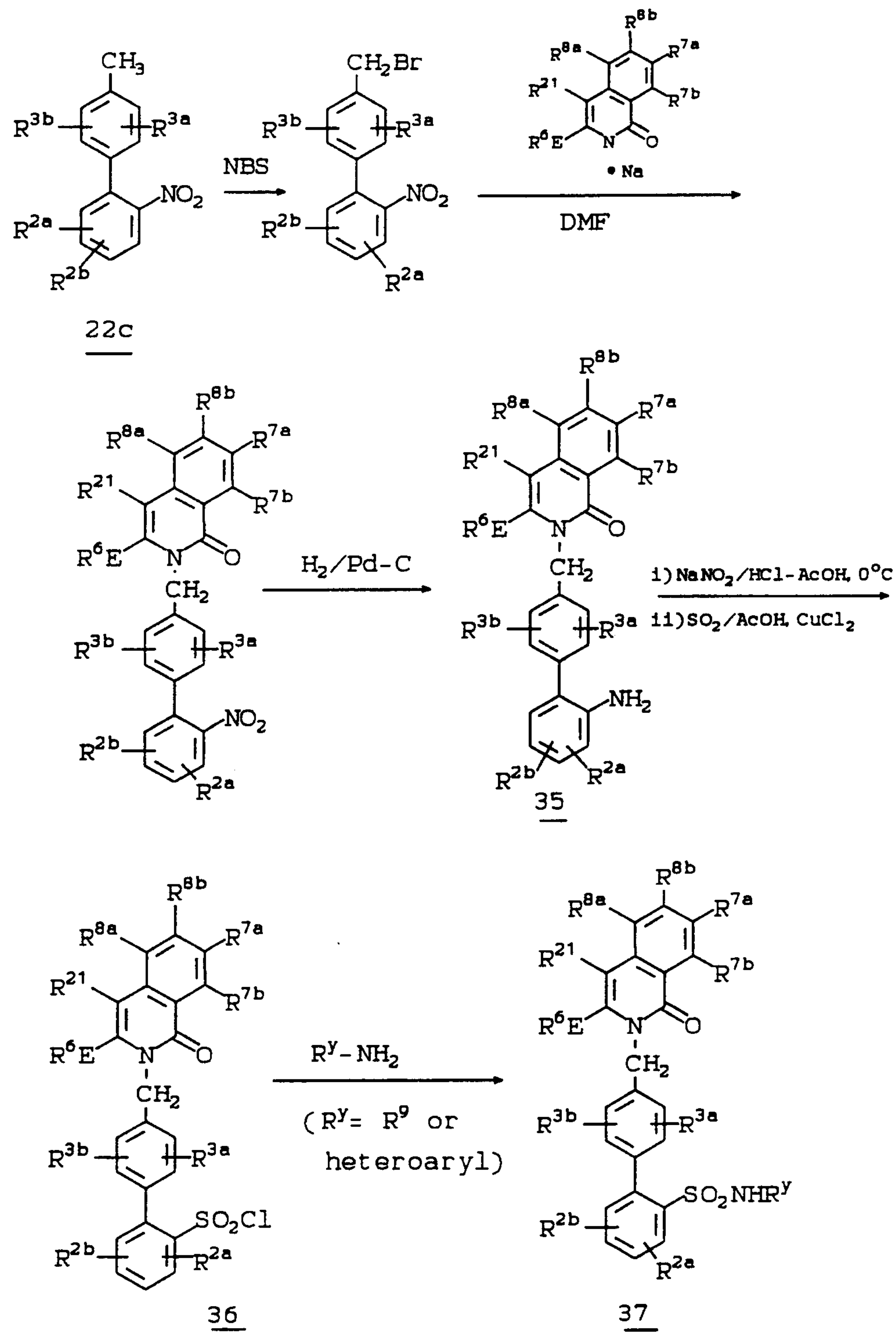


22c

35

36

37

The biaryl sulfonamides 38 and 39 (described in Scheme 9 as 31) can be prepared alternatively using palladium(O) catalyzed cross-coupling reactions of appropriate aryl-organotin precursors [J.K. Stille, Pure Appl. Chem., 57, 1771 (1985); T.R. Baiely, Tetrahedron Lett., 27, 4407 (1986); D.A. Widdowson and Y.Z. Zhang, Tetrahedron, 42, 2111 (1986)], as outlined in Scheme 11. The organotin compound 40 [S.M.

Moerlein, J. Organometallic Chem., 319, 29 (1987)], obtained from the aromatic precursor 41, may be coupled with aryl sulfonamide 42 and 43 using $Pd(PPh_3)_4$ or $(PPh_3)_2PdCl_2$ as catalysts to give biaryl sulfonamide 38 and 39. Similarly, the benzyl bromide 44 may be alternatively prepared from the appropriate organotin precursor 45 using the Pd(O) catalyzed cross-coupling reaction as outlined in Scheme 12.

SCHEME 11

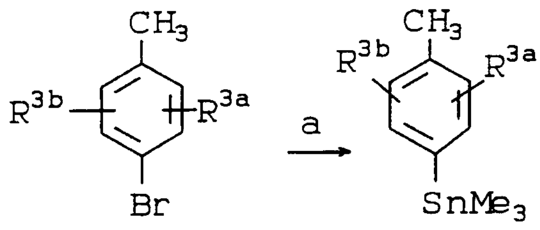


41 40

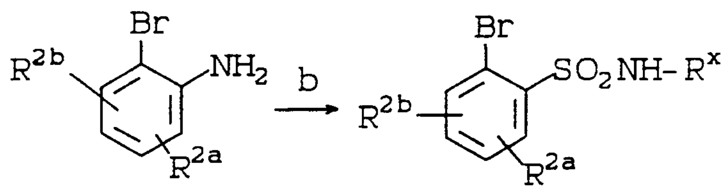


42 ($R^x$=-C($CH_3$)$_3$)

43 ($R^x$=-C($C_6H_5$)$_3$

40+(42 or 43)

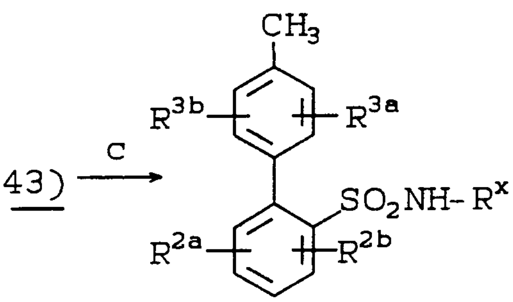


38 ($R^x$=-C($CH_3$)$_3$)

39 ($R^x$=-C($C_6H_5$)$_3$)

a.
  (i) t-BuLi/ether, -78° C
  (ii) $Me_3SnCl$
b.
  (i) $NaNO_2$/HCl
  (ii) $SO_2$, $CuCl_2$
c. $Pd(PPh_3)_4$, toluene, reflux or $(PPh_3)_2PdCl_2$, DMF, 90° C.

SCHEME 12

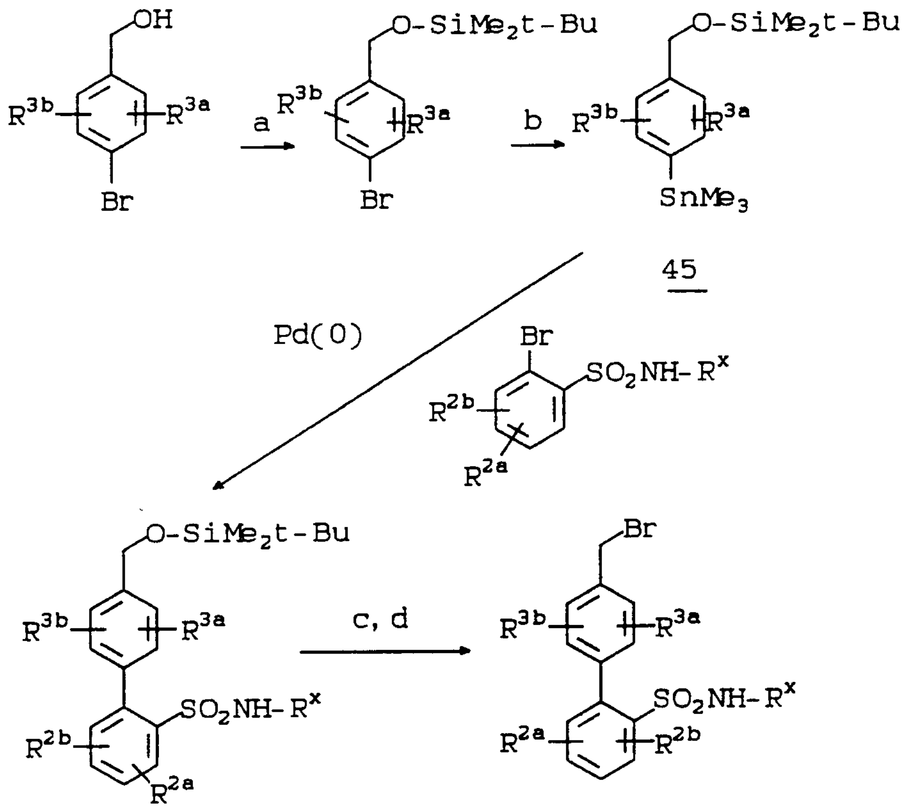


45

44(a) [$R^x$=-$C(C_6H_5)_3$]

44(b) [$R^x$=-$C(CH_3)_3$]

a. t-$BuMe_2$Si-Cl/imidazole, DMF
b. t-BuLi, -78°C, $Me_3SnCl$
c tetrabutylammonium fluoride
d. $CBr_4/Ph_3P$.

The compounds bearing $R^1$ = -$CH_2SO_2NHCOR^{22}$ and -$CH_2SO_2NHR^{22}$ may be prepared as outlined in Scheme 13. The key precursor aryl-methanesulfonyl chloride 46 may be prepared either from the reaction of arylmethylmagnesium chloride 47, obtained from the corresponding benzyl chloride 48 and magnesium, or by oxidation of the aryl-methylthioacetate 49 (prepared from the benzyl bromide 50 with chlorine in presence of trace amount of water) [Bagnay and Dransch, Chem. Ber., 93, 784 (1960)]. Alternatively, the aryl-methylthioacetate 49 can be oxidized with sulfuryl chloride in presence of acetic anhydride to form arylmethylsulfinyl chloride [S. Thea and G. Cevasco, Tet. Lett., 28, 5193 (1987)], which can be further oxidized with appropriate oxidizing agents to give the sulfonyl chloride 46. The compounds 51 and 52 can be obtained by reacting the sulfonyl chloride 46 with appropriate amines.

SCHEME 13

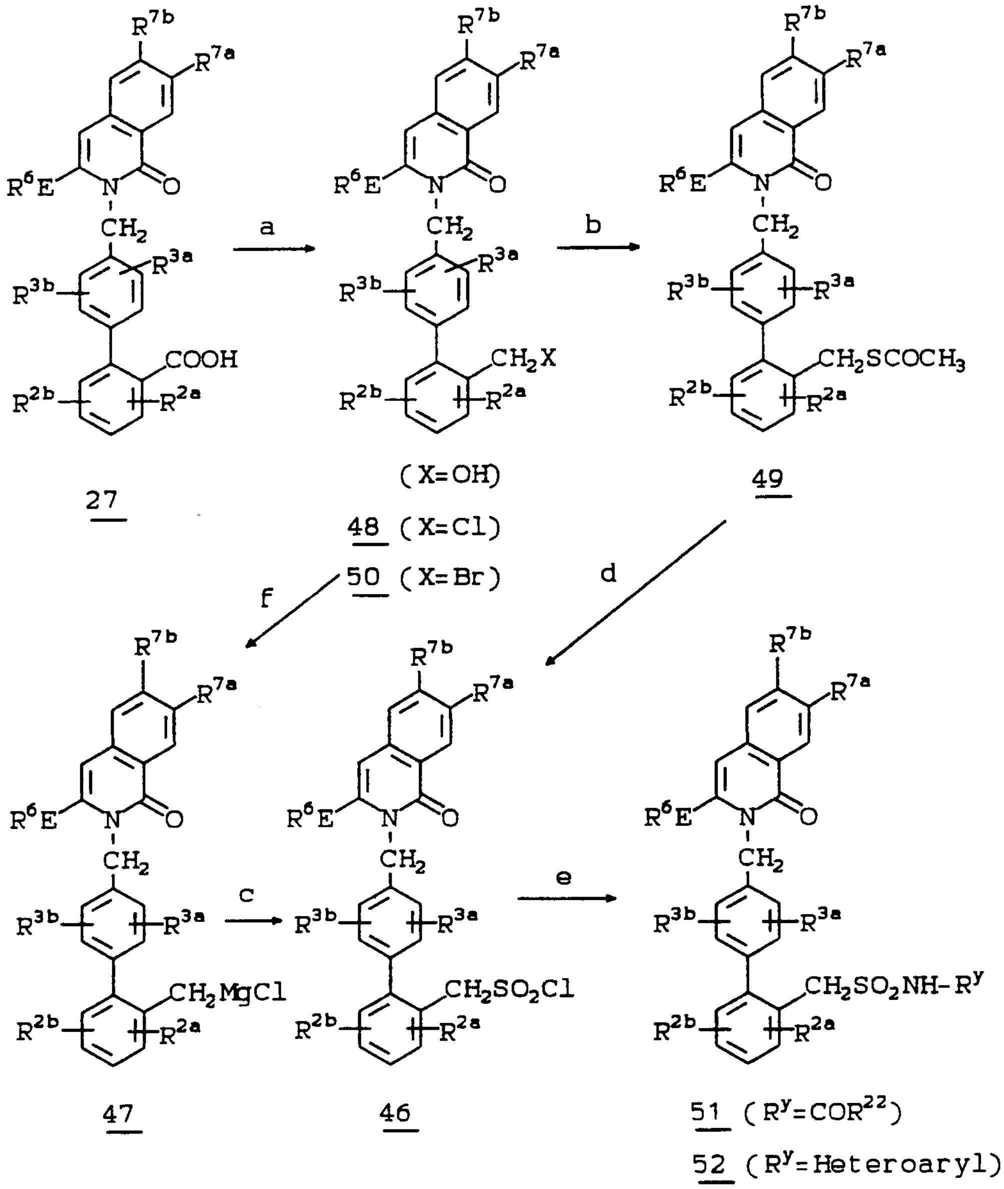


27

(X=OH)
48 (X=Cl)
50 (X=Br)

49

47

46

51 ($R^y$=$COR^{22}$)
52 ($R^y$=Heteroaryl)

a.
(i) $EtOCOCl/Et_3N$, THF, 0 ° C
(ii) $NaBH_4$
(iii) $CCl_4$ or $CBr_4/PPh_3$
b. AcSK
c. $SO_2Cl_2$
d. $Cl_2$, AcOH, $H_2O$ or,
(i) $SO_2Cl_2$
(ii) oxidation
e. $R^yNH_2$ or,
(i) $NH_3$
(ii) acylation.

Compounds where $R^1$ = $-NHSO_2NHR^{22}$ may be prepared by the reaction of appropriate primary amines with the sulfamide 53 [S.D. McDermott and W.J. Spillane, Synthesis, 192 (1983)], as described in Scheme 14. The compound 53 may be obtained from the corresponding N-t-butylsulfamide 54 after treatment with anhydrous trifluoroacetic acid [J.D. Catt and W.L. Matier, J. Org. Chem., 39, 566 (1974)]. The N-t-butylsulfamide 54 may be prepared by the reaction of the aromatic amine 55 (prepared as in Scheme 10)

with t-butylsulfamoyl chloride [W.L. Matier, W.T. Comer and D. Deitchman, J. Med. Chem., 15, 538 (1972)].

SCHEME 14

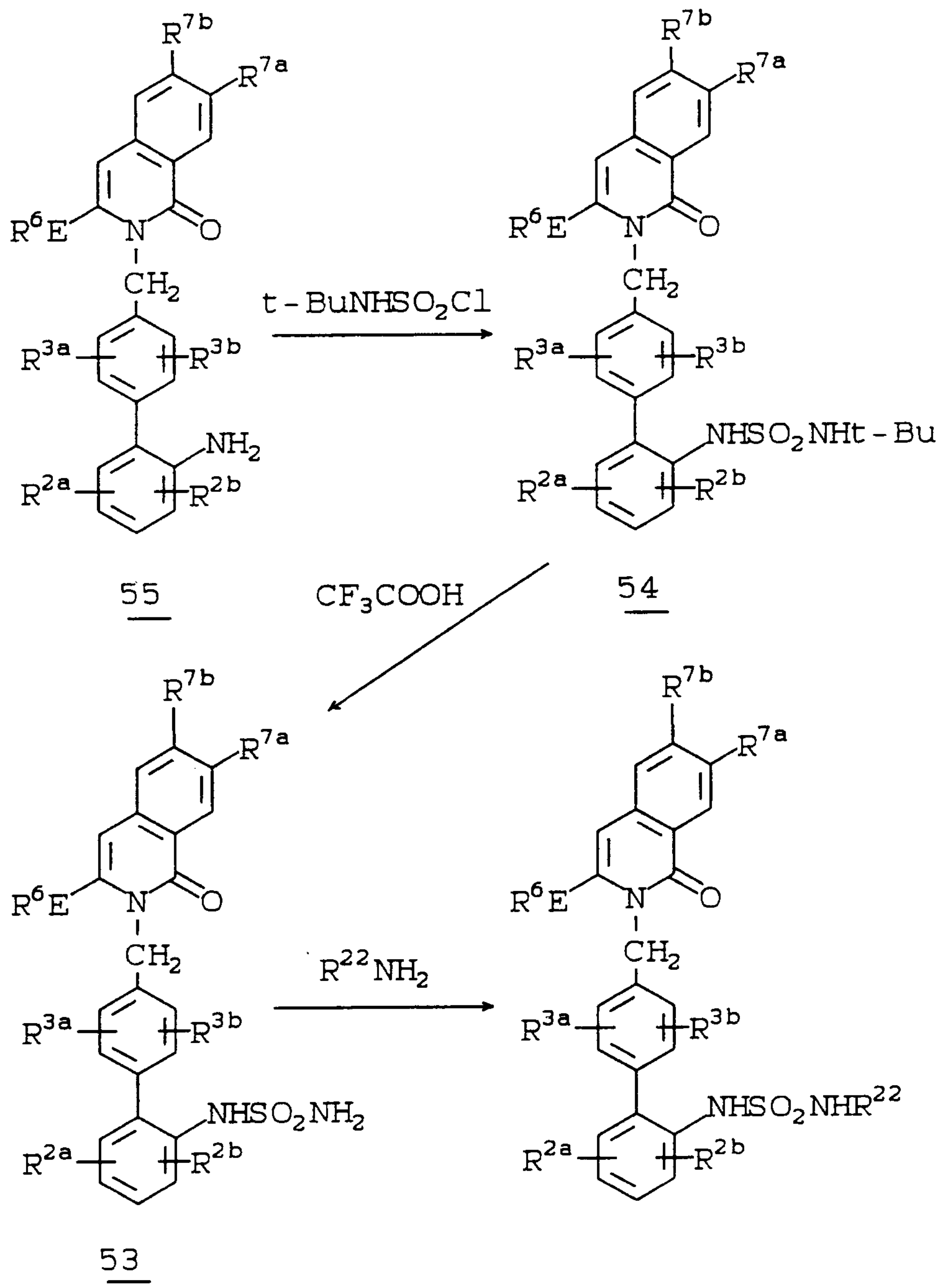


55

54

53

Further functionalization of compounds of Formula 1 where $R^{7a}$, $R^{7b}$, $R^{8a}$ or $R^{8b}$ is nitro is available through the following route (Scheme 15). The nitro group of 56 may be reduced to the amine 57 by reduction with hydrogen over palladium on carbon. The amine may then be acylated with acid chlorides to give amides under basic conditions. The acylation of the amine with chloroformates is best carried out in the presence of sodium hydride to form the anilinium anion. This anion reacts quickly with chloroformates to give the carbamates 58. The carbamate may be isolated and then deprotonated with lithium hexamethyldisilazide and alkylated to give the N,N-dialkylated carbamates 59. Alternatively this process may be carried out in one pot by first preforming the anilinium anion, acylating it and then deprotonating in situ and alkylating with $R^4$ iodide group to give 59. The amine 57 reacts slowly with isocyanates to give ureas 60.

Trisubstituted ureas 61 may be prepared from the benzyl carbamate 58 ($R^{22}$ = benzyl) by treatment with the magnesium salt of a secondary amine. The trisubstituted ureas may be N-alkylated by deprotonation with lithium hexamethyldisilazide and alkylation with an $R^4$ iodide to give 62. The amine may be further derivatized or converted to other groups by means of chemical procedures well known to those skilled in the art. Alternative $R^{22a}$ groups may be converted from intermediates prepared using the above protocol. The methods of carrying out these conversions are well known to those skilled in the art.

Related amides may be prepared by acylation of compound 57 to give the primary amide 63 which may then be deprotected to give a compound of structure 1, or alternatively alkylated under basic conditions (NaOH, $K_2CO_3$, phase transfer catalyst) to give a trisubstituted amide of structure 64.

SCHEME 15

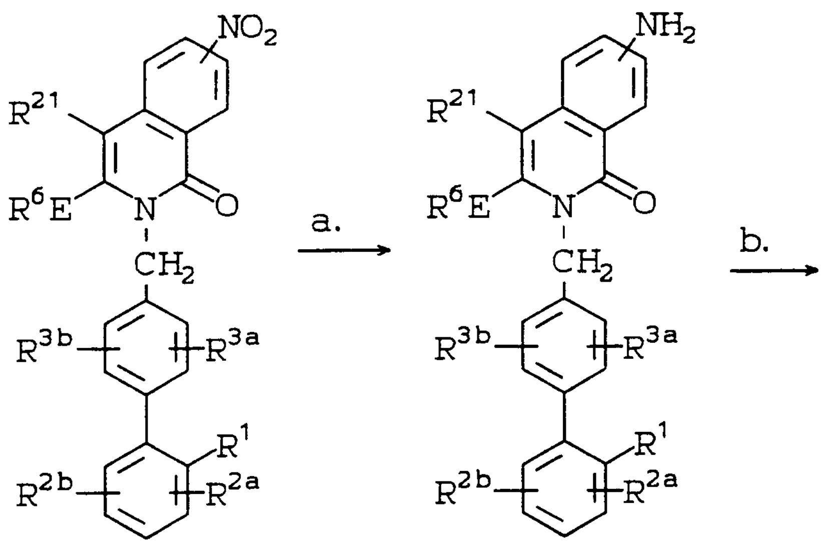


56 57

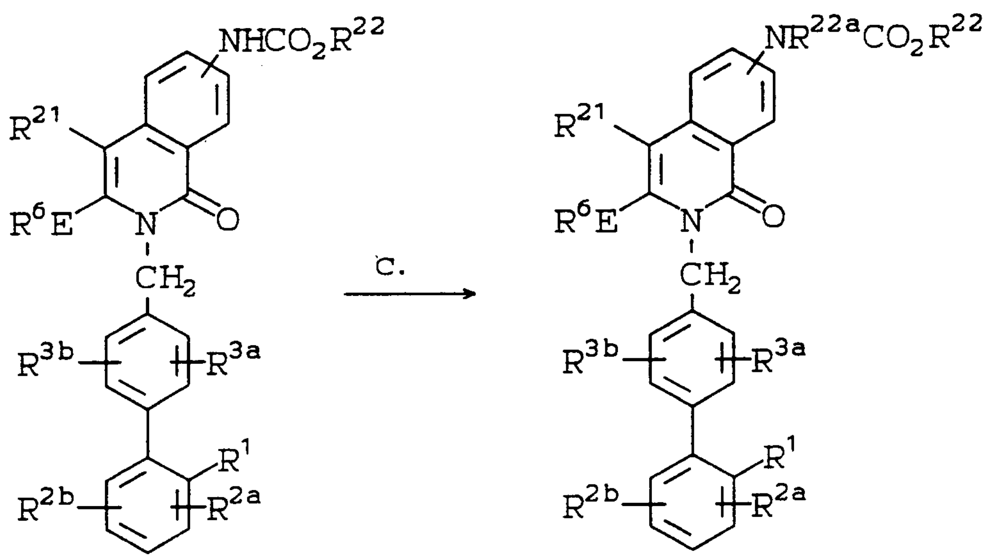


58 59

57 58

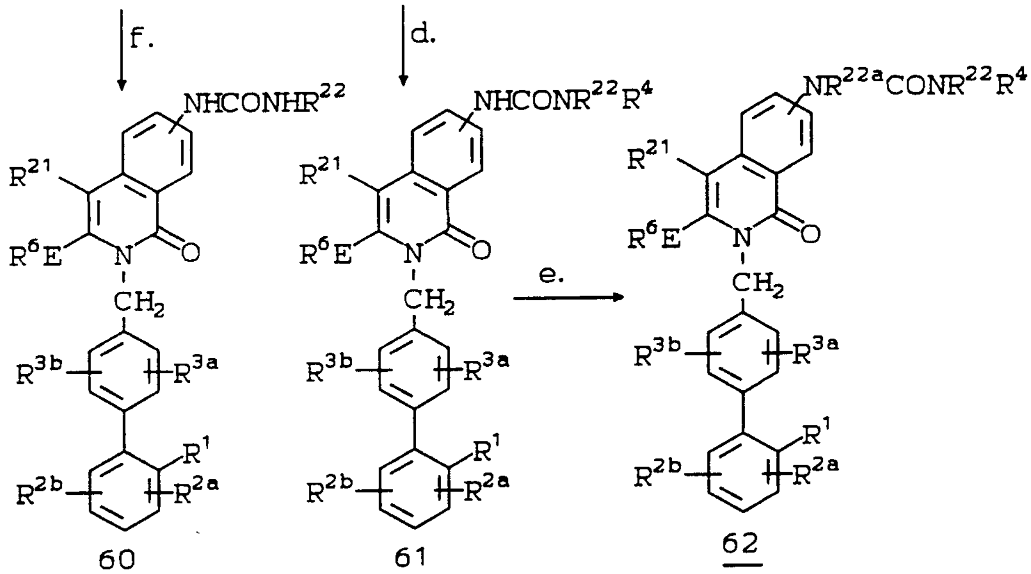


60 61 62

57

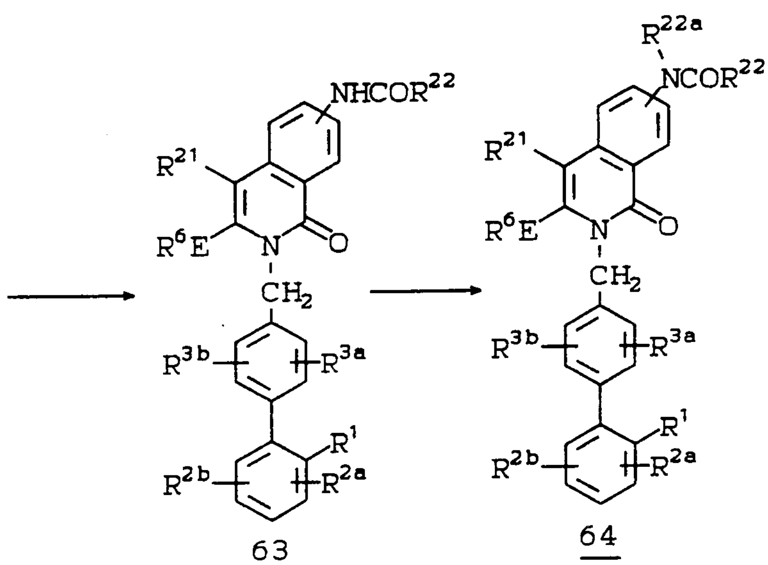

63 64

a. $H_2$, 10% Pd/C, EtAc; b. NaH, $ClCO_2R^{22}$, DMF;
c. $LiN(TMS)_2$, $R^{22a}I$;
d. MeMgBr, $R^4NHR^{22}$, THF, reflux;
e. $LiN(TMS)_2$, $R^{22a}I$, DMF; f. $R^{22}NCO$, $CH_2Cl_2$

Compounds wherein $R^1$ = $PO(OR^5)_2$ or $PO(R^9)(OR^5)$ may be prepared as indicated in Scheme 16. Coupling of compound 26 with a dibromoaryl will give compound 60. The aryl bromide 60 may be converted to an aryl phosphonic acid ester 61 or phosphinate 62. These may in turn be brominated under free radical conditions and then attached to the requisite isoquinolinone ring as shown in Scheme 1.

SCHEME 16

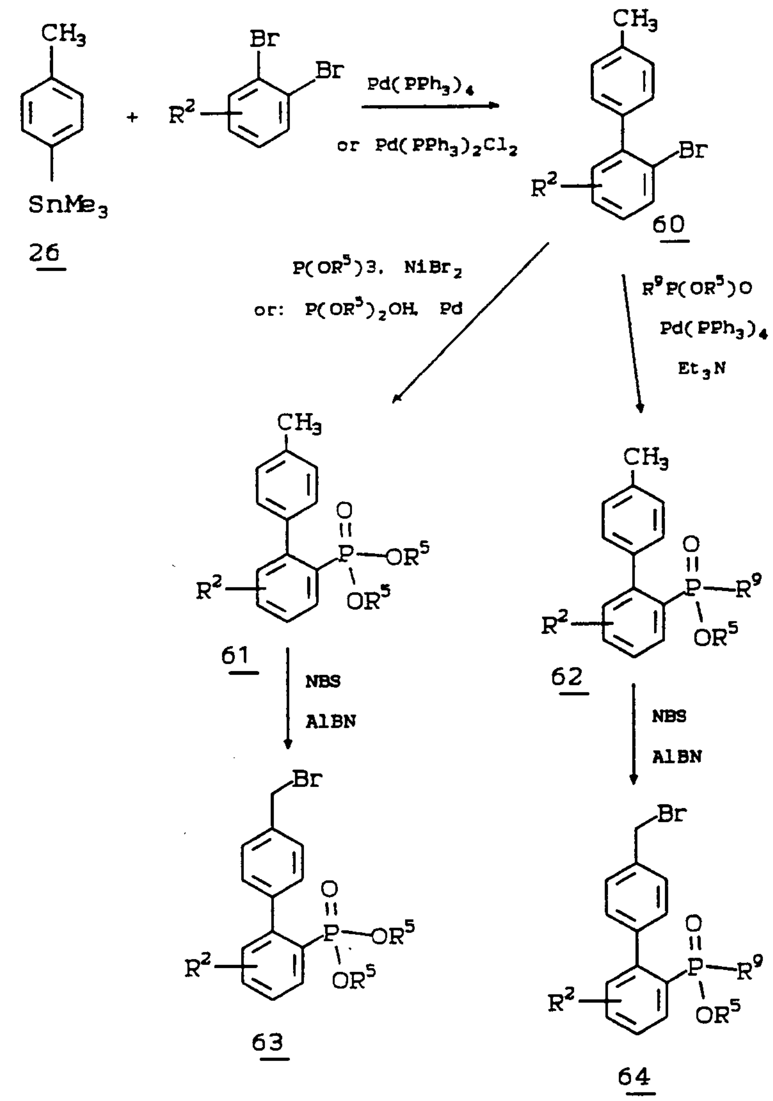


26 60 61 62 63 64

It will be appreciated by those skilled in the art that functional group transformations can be conducted on aryl and heterocyclic rings to afford desired analogs. For example, esters may be converted to amides by heating them with amines and an amide nitrogen if present in the heterocycle may be alkylated using bases such as sodium hydride in DMF with the appropriate alkyl halide. Functional group protection throughout these syntheses will be chosen to be compatible with subsequent reaction conditions. Ultimately such protecting groups will be removed to generate the desired optimally active compounds of Formula I. For example, $R^1$ as carboxyl is often protected as its t-butyl ester which in the last step is removed by treatment with trifluoroacetic acid. Aqueous acetic acid at room temperature overnight is a preferred method to remove a trityl protecting group to liberate an $R^1$ tetrazole group.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases; e.g., dicyclohexylamine salts, N-methyl-D-glucamine, salts with amino acids like arginine, lysine, and the like. Also, salts with organic and inorganic acids may be prepared; e.g., HCl, HBr, $H_2SO_4$, $H_3PO_4$, methane-sulfonic, toluene-sulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed in vacuo or by freeze-drying or by

exchanging the cations of an existing salt for another cation on a suitable ion exchange resin.

Angiotensin II (AII) is a powerful arterial vasoconstrictor, and it exerts its action by interacting with specific receptors present on cell membranes. The compounds described in the present invention act as competitive antagonists of AII at the receptors. In order to identify AII antagonists and determine their efficacy in vitro, the following two ligand-receptor binding assays were established.

Receptor binding assay using rabbit aortae membrane preparation:

Three frozen rabbit aortae (obtained from Pel-Freeze Biologicals) were suspended in 5mM Tris-0.25M Sucrose, pH 7.4 buffer (50 ml) homogenized, and then centrifuged. The mixture was filtered through a cheesecloth and the supernatant was centrifuged for 30 minutes at 20,000 rpm at 4° C. The pellet thus obtained was resuspended in 30 ml of 50mM Tris-5 mM $MgCl_2$ buffer containing 0.2% Bovine Serum Albumin and 0.2 mg/ml Bacitration and the suspension was used for 100 assay tubes. Samples tested for screening were done in duplicate. To the membrane preparation (0.25 ml) there was added $^{125}$I-Sar$^1$Ile$^8$-angiotensin II [obtained from New England Nuclear] (10μl; 20,000 cpm) with or without the test sample and the mixture was incubated at 37° C for 90 minutes. The mixture was then diluted with ice-cold 50mM Tris-0.9% NaCl, pH 7.4 (4ml) and filtered through a glass fiber filter (GF/B Whatman 2.4" diameter). The filter was soaked in scintillation cocktail (10 ml) and counted for radioactivity using Packard 2660 Tricarb liquid scintillation counter. The inhibitory concentration ($IC_{50}$) of potential AII antagonist which gives 50% displacement of the total specifically bound $^{125}$I-Sar$^1$Ile$^8$-angiotensin II was presented as a measure of the efficacy of such compounds as AII antagonists.

Receptor assay using Bovine adrenal cortex preparation

Bovine adrenal cortex was selected as the source of AII receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added 3H-angiotensin II (50mM) (10μl) with or without the test sample and the mixture was incubated at 37° C for 1 hour. The mixture was then diluted with Tris buffer (4ml) and filtered through a glass fiber filter (GF/B Whatman 2.4" diameter). The filter was soaked in scintillation cocktail (10ml) and counted for radioactivity using Packard 2660 Tricarb liquid scintillation counter. The inhibitory concentration ($IC_{50}$) of potential AII antagonist which gives 50% displacement of the total specifically bound $^3$H-angiotensin II was presented as a measure of the efficacy of such compounds as AII antagonists.

Receptor assay using rat brain membrane preparation

Membranes from rat brain (thalamus, hypothamus and midbrain) were prepared by homogenization in 50 mM Tris HCl (pH 7.4), and centrifuged at 50,000 x g. The resulting pellets were washed twice in 100 mM NaCl, 5 mM $Na_2$•EDTA, 10 mM $Na_2HPO_4$ (pH 7.4) and 0.1 mM PMSF by resuspension and centrifugation. For binding assays, the pellets were resuspended in 160 volumes of binding assay buffer (100 mM NaCl, 10 mM $Na_2HPO_4$, 5 mM $Na_2$•EDTA, pH 7.4, 0.1 mM PMSF, 0.2 mg/ml soybean trypsin inhibitor, 0.018 mg/ml o-phenanthroline, 77 mg/ml dithiothreitol and 0.14 mg/ml bacitracin. For $^{125}$I.Ile$^8$-angiotensin II binding assays, 10 μl of solvent (for total binding), Sar$^1$,Ile$^8$-angiotensin II (1 μM) (for nonspecific binding) or test compounds (for displacement) and 10 μl of [$^{125}$I]Sar$^1$,Ile$^8$-angiotensin II (23-46 pM) were added to duplicate tubes. The receptor membrane preparation (500 μl) was added to each tube to initiate the binding reaction. The reaction mixtures were incubated at 37° C for 90 minutes. The reaction was then terminated by filtration under reduced pressure through glass-fiber GF/B filters and washed immediately 4 times with 4 ml of 5 mM ice-cold Tris HCl (pH 7.6) containing 0.15 M NaCl. The radioactivity trapped on the filters was counted using a gamma counter.

Using the methodology described above, representative compounds of this invention were evaluated and were found to exhibit an activity of at least $IC_{50}<50$ μM, thereby demonstrating and confirming the utility of the compounds of the invention as effective A II antagonists.

The antihypertensive effects of the compounds described in the present invention may be evaluated using the methodology described below:

Male Charles River Sprague-Dawley rats (300-375 gm) were anesthetized with methohexital (Brevital; 50

mg/kg i.p.) and the trachea was cannulated with PE 205 tubing. A stainless steel pithing rod (1.5 mm thick, 150 mm long) was inserted into the orbit of the right eye and down th spinal column. The rats were immediately placed on a Harvard Rodent Ventilator (rate - 60 strokes per minute, volumn - 1.1 cc per 100 grams body weight). The right carotid artery was ligated, both left and right vagal nerves were cut, and the left carotid artery was cannulated with PE 50 tubing for drug administration, and body temperature was maintained at 37 °C by a thermostatically controlled heating pad which received input from a rectal temperature probe. Atropine (1 mg/kg i.v.) was then administered, and 15 minutes later propranolol (1 mg/kg i.v.). Thirty minutes later antagonists of the Formula I were administered intravenously or orally. Angiotensin II was then typically given at 5, 10, 15, 30, 45 and 60 minute intervals and every half hour thereafter for as long as the test compound showed activity. The change in the mean arterial blood pressure was recorded for each angiotensin II challenge and the percent inhibition of the angiotensin II response was calculated.

The compounds of the invention are useful in treating hypertension. They are also of value in the management of acute and chronic congestive heart failure. These compounds may also be expected to be useful in the treatment of secondary hyperaldosteronism, primary and secondary pulmonary hyperaldosteronism, primary and secondary pulmonary hypertension, renal failure such as diabetic nephropathy, glomerulonephritis, scleroderma, glomerular sclerosis, proteinuria of primary renal disease, end stage renal disease, renal transplant therapy, and the like, renal vascular hypertension, left ventricular dysfunction, diabetic retinopathy and in the management of vascular disorders such as migraine, Raynaud's disease, luminal hyperclasia, and to minimize the atherosclerotic process. The application of the compounds of this invention for these and similar disorders will be apparent to those skilled in the art.

The compounds of this invention are also useful to treat elevated intraocular pressure and to enhance retinal blood flow and can be administered to patients in need of such treatment with typical pharmaceutical formulations such as tablets, capsules, injectables and the like as well as topical ocular formulations in the form of solutions, ointments, inserts, gels, and the like. Pharmaceutical formulations prepared to treat intraocular pressure would typically contain about 0.1% to 15% by weight, preferably 0.5% to 2% by weight, of a compound of this invention.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg. per patient per day which can be administered in single or multiple doses. Perferably, the dosage range will be about 2.5 to 250 mg. per patient per day; more preferably about 5 to 150 mg. per patient per day.

The compounds of this invention can also be administered in combination with other antihypertensives and/or diuretics and/or angiotensin converting enzyme inhibitors and/or calcium channel blockers. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, and the like, as well as admixtures and combinations thereof.

The useful central nervous system (CNS) activities of the compounds of this invention are demonstrated and exemplified by the ensuing assays.

## COGNITIVE FUNCTION ASSAY

The efficacy of these compounds to enhance cognitive function can be demonstrated in a rat passive avoidance assay in which cholinomimetics such as physostigmine and nootropic agents are known to be active. In this assay, rats are trained to inhibit their natural tendency to enter dark areas. The test apparatus used consists of two chambers, one of which is brightly illuminated and the other is dark. Rats are placed in

the illuminated chamber and the elapsed time it takes for them to enter the darkened chamber is recorded. On entering the dark chamber, they receive a brief electric shock to the feet. The test animals are pretreated with 0.2 mg/kg of the muscarinic antagonist scopolamine which disrupts learning or are treated with scopolamine and the compound which is to be tested for possible reversal of the scopolamine effect. Twenty-four hours later, the rats are returned to the illuminated chamber. Upon return to the illuminated chamber, normal young rats who have been subjected to this training and who have been treated only with control vehicle take longer to re-enter the dark chamber than test animals who have been exposed to the apparatus but who have not received a shock. Rats treated with scopolamine before training do not show this hesitation when tested 24 hours later. Efficacious test compounds can overcome the disruptive effect on learning which scopolamine produces. Typically, compounds of this invention should be efficacious in this passive avoidance assay in the dose range of from about 0.1 mg/kg to about 100 mg/kg.

ANXIOLYTIC ASSAY

The anxiolytic activity of the invention compounds can be demonstrated in a conditioned emotional response (CER) assay. Diazepam is a clinically useful anxiolytic which is active in this assay. In the CER protocol, male Sprague-Dawley rats (250-350 g) are trained to press a lever on a variable interval (VI) 60 second schedule for food reinforcement in a standard operant chamber over weekly (five days per week) training sessions. All animals then receive daily 20 minute conditioning sessions, each session partitioned into alternating 5 minute light (L) and 2 minute dark (D) periods in a fixed L1D1L2D2L3 sequence. During both periods (L or D), pressing a lever delivers food pellets on a VI 60 second schedule: in the dark (D), lever presses also elicit mild footshock (0.8 mA, 0.5 sec) on an independent shock presentation schedule of VI 20 seconds. Lever pressing is suppressed during the dark periods reflecting the formation of a conditioned emotional response (CER).

Drug testing in this paradigm is carried out under extinction conditions. During extinction, animals learn that responding for food in the dark is no longer punished by shock. Therefore, response rates gradually increase in the dark periods and animals treated with an anxiolytic drug show a more rapid increase in response rate than vehicle treated animals. Compounds of this invention should be efficacious in this test procedure in the range of from about 0.1 mg/kg to about 100 mg/kg.

DEPRESSION ASSAY

The antidepressant activity of the compounds of this invention can be demonstrated in a tail suspension test using mice. A clinically useful antidepressant which serves as a positive control in this assay is desipramine. The method is based on the observations that a mouse suspended by the tail shows alternate periods of agitation and immobility and that antidepressants modify the balance between these two forms of behavior in favor of agitation. Periods of immobility in a 5 minute test period are recorded using a keypad linked to a microcomputer which allows the experimenter to assign to each animal an identity code and to measure latency, duration and frequency of immobile periods. Compounds of this invention should be efficacious in this test procedure in the range of from about 0.1 mg/kg to about 100 mg/kg.

SCHIZOPHRENIA ASSAY

The antidopaminergic activity of the compounds of this invention can be demonstrated in an apomorphine-induced sterotypy model. A clinically useful antipsychotic drug that is used as a positive control in this assay is haloperidol. The assay method is based upon the observation that stimulation of the dopaminergic system in rats produces stereo-typed motor behavior. There is a strong correlation between the effectiveness of classical neuroleptic drugs to block apomorphine-induced stereotypy and to prevent schizophrenic symptoms. Stereotyped behavior induced by apomorphine, with and without pretreatment with test compounds, is recorded using a keypad linked to a microcomputer. Compounds of the invention should be efficacious in this assay in the range of from about 0.1 mg/kg to about 100 mg/kg.

In the treatment of the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will

recognize, the dosage range will generally be about 5 to 6000 mg. per patient per day which can be administered in single or multiple doses. Perferably, the dosage range will be about 10 to 4000 mg. per patient per day; more preferably about 20 to 2000 mg. per patient per day.

In order to obtain maximal enhancement of cognitive function, the compounds of this invention may be combined with other cognition-enhancing agents. These include acetylcholinesterase inhibitors such as heptylphysostigmine and tetrahydroacridine (THA; tacrine), muscarinic agonists such as oxotremorine, inhibitors of angiotensin-converting enzyme such as octylramipril, captopril, ceranapril, enalapril, lisinopril, fosinopril and zofenopril, centrally-acting calcium channel blockers and as nimodipine, and nootropic agents such as piracetam.

In order to achieve optimal anxiolytic activity, the compounds of this invention may be combined with other anxiolytic agents such as alprazolam, lorazepam, diazepam, and busipirone.

In order to achieve optimal antidepressant activity, combinations of the compounds of this invention with other antidepressants are of use. These include tricyclic antidepressants such as nortriptyline, amitryptyline and trazodone, and monoamine oxidase inhibitors such as tranylcypromine.

In order to obtain maximal antipsychotic activity, the compounds of this invention may be combined with other antipsychotic agents such as promethazine, fluphenazine and haloperidol.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 2.5-250 milligrams per day range can be effectively combined at levels at the 0.5-250 milligrams per day range with the following compounds at the indicated per day dose range: hydrochlorothiazide (15-200 mg) chlorothiazide (125-2000 mg), ethacrynic acid (15-200 mg), amiloride (5-20 mg), furosemide (5-80 mg), propranolol (20-480 mg), timolol maleate (5-60 mg.), methyldopa (65-2000 mg), felodipine (5-60 mg), nifedipine (5-60 mg), and nitrendipine (5-60 mg). In addition, triple drug combinations of hydrochlorothiazide (15-200 mg) plus amiloride (5-20 mg) plus angiotensin II antagonist of this invention (3-200 mg) or hydrochlorothiazide (15-200 mg) plus timolol maleate (5-60) plus an angiotensin II antagonist of this invention (0.5-250 mg) or hydrochlorothiazide (15-200 mg) and nifedipine (5-60 mg) plus an angiotensin II antagonist of this invention (0.5-250 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the unit dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The following examples further illustrate the preparation of the compounds of Formula I and their incorporation into pharmaceutical compositions and, as such, are not to be considered or construed as limiting the invention recited in the appended claims.

## EXAMPLE 1

3-Butyl-2-[(2'-(tetrazole-5-yl)biphen-4-yl)methyl]-1(2H)-isoquinolinone

(a) 3-n-Butyl-1(2H)-isoquinolinone

To a solution of 4 g (24 mmol) of homophthalic anhydride in 12 ml of dry pyridine in a 250 L 3 neck flask fitted with a reflux condenser and addition funnel at 0°C was added 5.4 g (45 mmol) of valeryl chloride in 16 ml of dry chloroform over 45 minutes. The reaction mixture was stirred for a further 1 hour and then was treated with 100 ml of conc. ammonium hydroxide dropwise. The mixture was refluxed for 2 hours and then allowed to stand overnight at room temperature. The two phases were separated and the organic phase was washed with water (1 x 20 ml). The organic phase was dried over $MgSO_4$, filtered and concentrated in vacuo. The residue was recrystallized from hot MeOH/water to give a pale purple solid.
$^1$H-NMR ($CDCl_3$): 0.96 (t, 3H, J=7.2Hz), 1.45 (m, 2H), 1.73 (m, 2H), 2.64 (3 line m, 2H, J=7.8Hz), 6.32 (s, 1H), 7.37-7.67 (m, 3H), 8.37 (d, 1H, J=7.8Hz). FABMS: 202 ($M^+$ +1).

(b) 3-Butyl-2-[(2'-(N-triphenylmethyl-tetrazole-5-yl)biphen-4-yl)methyl]-1(2H)-isoquinolinone

To a suspension of 32.8 mg (1.09 mmol) of sodium hydride in 1.5 ml of dry DMF at 0°C was added 0.2 g (0.99 mmol) of 3-n-butyl-1(2H)-isoquinolinone as a solid. The solid dissolved with evolution of $H_2$ gas. After 1 hour 0.6 g (1.09 mmol) of N-triphenylmethyl-5-[2-(4'-bromomethylbiphenyl)]tetrazole was added dissolved in 2 ml of DMF. The reaction mixture was stirred overnight. The solution was diluted with 50 ml of EtOAc, washed with water (3 x 10 ml) and brine (1 x 10 ml), dried over $MgSO_4$ and concentrated in vacuo. The product was purified by flash chromatography over silica eluting with 20% EtOAc/hexanes to give a thick oil.
$^1$H-NMR ($CDCl_3$): 0.87 (t, 3H, J=7.3Hz), 1.28 (m, 2H), 2.46 (t, 2H), J=7.7Hz), 5.30 (bs, 2H), 6.89 (m, 8H), 7.03 (d, 2H, J=7.8Hz), 7.17-7.31 (m, 10H), 7.42 (m, 4H), 7.61 (t, 1H, J=7.6Hz), 7.85 (dd, 1H, J=1.8, 7.6Hz), 8.40 (dd, 1H, J=0.7, 8.1Hz).

(c) General Procedure for the Deprotection of the Tetrazole

The triphenyl methyl group was removed by dissolving, for example, 3-Butyl-2-[(2'-(N-triphenylmethyl-tetrazole-5-yl)biphen-4-yl)methyl]-1(2H)-isoquinolinone (0.33 g) in MeOH (5 ml) in the presence of several drops (3-5) of concentrated hydrochloride acid. After 2 hours at room temperature a few crystals of phenophthalien were added and the reaction mixture made basic by addition of 5N NaOH solution. The reaction mixture was reacidified by addition of acetic acid and then concentrated in vacuo. The residue was dissolved in 20 ml of EtOAc and washed with water (3 x 5 ml) and brine (1 x 5 ml) and dried over $MgSO_4$. The mixture was filtered and concentrated in vacuo and the residue was purified by flash chromatography over silica gel eluting with the solvent indicated below.

3-Butyl-2-[(2'-(tetrazole-5-yl)biphen-4-yl)methyl]-1(2H)-isoquinolinone

Purified by elution with 50% EtOAc/hexanes 1% acetic acid.
$^1$H-NMR ($CDCl_3$): 0.91 (t, 3H, J=7.4Hz), 1.39 (m, 2H), 1.62 (m, 2H), 2.60 (3 line m, 7.4Hz), 5.33 (bs, 2H), 6.44 (s, 1H), 7.02 (ABq, 4H, J=8.0Hz), 7.35 (m, 2H), 7.36 (m, 2H), 7.54 (t, 1H, J=7.7Hz), 7.61 (t, 1H, J=7.0Hz), 7.97 (bm, 1H), 8.17 (d, 1H, J=8.0Hz). FABMS=436 ($M^+$ +1).

EXAMPLE 2

7-(N-Methyl-N-isobutyloxycarbonyl)amino-3-propyl-2-[(2'-(tetrazole-5-yl)biphen-4-yl)methyl]-1(2H)-isoquinolinone

(a) 7-Nitro-3-propyl-1(2H)-isoquinolinone

To a mixture of 50 ml of butyric anhydride and 10 ml of pyridine in a dry flask was added 10 g (0.45 mol) of 4-nitrohomophthalic anhydride (prepared as in J. Organic Chemistry, 533, 1945). A thick red suspension formed which prevented efficient stirring. 50 ml of ether was added to facilitate stirring. After 3 hours the reaction mixture was filtered and the precipitate was washed with ether. The residue was dried under vacuum to give 15.3 g of a red solid. The solid was suspended in 250 ml of ammonium hydroxide and heated to 80°C for 3 hours. Further 20 ml portions of ammonium hydroxide were added at 45 minute

intervals. The mixture was cooled to room temperature and filtered. The residue was recrystallized from hot ethanol to give yellow crystals.
$^1$H-NMR ($CDCl_3$): 1.04 (t, 3H, J=7.4Hz), 1.79 (m, 2H), 2.62 (3 line m, 2H, J=7.6Hz), 6.37 (s, 1H), 7.57 (d, 1H, J=8.8Hz), 8.39 (dd, 1H, J=2.4, 8.8Hz), 9.20 (d, 1H, J=2.1Hz).

(b) 7-Nitro-3-propyl-2-[(2'-(N-triphenylmethyl-tetrazole-5-yl)biphen-4-yl)methyl]-1(2H)-isoquinolinone

To a suspension of 0.377 g (0.0157 mol) of sodium hydride in 20 ml of dry DMF at 0°C was added 3.32 g (0.0143 mol) of 7-nitro-3-propyl-1(2H)-isoquinolinone as a solid. Some hydrogen evolution occured with formation of a deep red suspension. Addition of a further 10 ml of DMF failed to give complete solvation. After 30 minutes at room temperature the reaction mixture was cooled to 0°C and 8.74 g (0.0157 mol) of N-triphenylmethyl-5-[2-(4'-bromomethylbiphenyl)]tetrazole dissolved in 40 ml of dry DMF was added. The reaction mixture was stirred overnight at room temperature and poured into 300 ml of water. The suspension was filtered and the solid residue dissolved in 200 ml of $CH_2Cl_2$. The solution was washed with brine (2 x 20 ml) and dried over $MgSO_4$, filtered and concentrated in vacuo. The residue was purified by flash chromatography over silica gel eluting with 50% EtOAc/hexanes to give a foam. This material was contaminated with a regioisomer to the extent of 10%.
$^1$H-NMR ($CDCl_3$): 0.83 (t, 3H), 1.62 (m, 2H), 2.50 (t, 2H), 5.31 (bs, 2H), 6.39 (s, 1H), 6.89 (m, 8H), 7.05 (d, 2H), 7.17-7.31 (m, 10H), 7.41 (m, 2H), 7.55 (d, 1H), 7.87 (d, 1H), 7.38 (dd, 1H), 9.25 (d, 1H).

(c) 7-Amino-3-propyl-2-[(2'-(N-triphenylmethyl-tetrazole-5-yl)biphen-4-yl)methyl]-1(2H)-isoquinolinone

A solution of 1.45 g (2.04 mmol) of 7-Nitro-3-propyl-2-[(2'-(N-triphenylmethyl-tetrazole-5-yl)biphen-4-yl)-methyl]-1(2H)-isoquinolinonedissolved in 50 ml of EtOAc was hydrogenated at atmospheric pressure over 0.2 g of 10% Pd/C overnight. The reaction mixture was filtered through celite and the filtrate was concentrated in vacuo. The residue was purified by flash chromatography over silica gel eluting with 50% EtOAc/hexanes to give the pure amine as a foam.
$^1$H-NMR ($CDCl_3$): 0.87 (t, 3H, J=7.36Hz), 1.56 (m, 2H), 2.39 (3 line m, 2H, J=7.7Hz), 5.29 (bs, 2H), 6.24 (s, 1H), 6.91 (d, 4H, J=7.9Hz), 7.03 (m, 2H), 7.19-7.22 (m, 10H), 7.42 (m, 2H), 7.63 (d, 1H, J=2.4Hz), 7.85 (dd, 1H, J=1.7, 7.4Hz).

(d) General Procedure for Preparation of Carbamates

To a suspension of 1.1 eq. of 80% NaH suspended in a volume of dry DMF that would make a 0.1 M solution was added at 0°C under $N_2$ a solution of 1.0 eq. of 7-amino-3-propyl-2-[(2'-(N-triphenylmethyl-tetrazole-5-yl)biphen-4-yl)methyl]-1(2H)-isoquinolinone dissolved in a minimal amount of DMF. After 30 minutes the chloroformate ($ClCOOR^{22}$) of choice was added neat. The reaction mixture was stirred for 30 minutes. To this mixture was added 1.2 eq of a 1M solution of lithium hexamethyldisilazide in THF. After 30 minutes at 0°C the alkylating agent ($R^{22a}$) was added neat and the reaction mixture was stirred overnight allowing the temperature to increase to room temperature. The reaction mixture was diluted with EtOAc (10 times the volume of DMF used) and brine. The solution was dried over $MgSO_4$, filtered and concentrated in vacuo. The residue was purified using the techniques described below for the specific examples.

7-(N-Methyl-N-isobutyloxycarbonyl)amino-3-propyl-2-[(2'-(N-triphenylmethyl-tetrazole-5-yl)biphen-4-yl)-methyl]-1(2H)-isoquinolinone

Purified by HPLC over a Lobar B silica gel column eluting with 30% EtOAc/hexanes to give a single product as a foam.
$^1$H-NMR ($CDCl_3$): 0.83-0.91 (m, 9H), 0.58 (m, 2H), 0.89 (m, 1H), 2.44 (3 line m, 2H, J=7.4Hz), 3.37 (s, 3H), 3.90 (d, 2H, J=6.6Hz), 5.29 (bs, 2H), 6.32 (s, 1H), 7.89 (m, 4H), 7.03 (d, 2H, J=8.2Hz), 7.19-7.31 (m, 10H), 7.42 (m, 3H), 7.61 (bm, 1H), 7.86 (dd, 1H, J-1.95, 7.3Hz), 8.20 (d, 1H, J=2.2Hz).

(e) 7-(N-Methyl-N-isobutyloxycarbonyl)amino-3-propyl-2-[(2'-(tetrazole-5-yl)biphen-4-yl)methyl]-1(2H)-isoquinolinone

Utilizing the General Procedure for the Deprotection of Tetrazole from Example 1(c), the above title compound was prepared from the compound from Example 2(d) and the purified by flash chromatography over silica gel eluting with 50% EtOAc/hexanes 1% acetic acid.

$^1$H-NMR ($CDCl_3$): 0.86 (d, 6H, J=6.02 Hz), 0.90 (t, 3H, J=7.04 Hz), 1.67 (m, 2H), 1.87 (m, 1H), 2.57 (3 line m, 2H, J=7.7 Hz), 3.32 (s, 3H), 3.38 (d, 2H, J=6.2Hz), 5.33 (bs, 2H); 6.42 (s, 1H); 7.02 (m, 4H); 7.15 (d, 1H, J=7 Hz); 7.22 (d, 1H, J=7 Hz), 7.35 (d, 1H, J=7.9 Hz); 7.43 (d, 1H, J=8.8 Hz); 7.45-7.67 (m, 2H); 8.03 (bs, 1H).

(f) 7-(N-Benzyl-N-isobutyloxycarbonyl)-amino-3-propyl-2-[(2'-(tetrazol-5-yl)biphen-4-yl)-methyl]-1(2H)-isoquinolinone

Prepared as described in the general procedure outlined in 2(d). Purified by flash chromatography over silica gel eluting with 40% EtOAc/hexanes. $^1$H-NMR ($CDCl_3$): 0.80 (d, 6H, J=6.9 Hz), 0.87 (t, 3H, J=7.6 Hz), 1.57 (m, 2H), 1.83 (m, 1H), 2.42 (3 line m, 2H, J=7.6 Hz), 3.91 (d, 3H, J=6.6 Hz), 7.16-7.49 (m, 19H), 7.87 (dd, 1H, J=1.5, 7.4 Hz), 8.24 (s, 1H).

(g) 7-(N-Benzyl-N-isobutyloxycarbonyl)-amino-3-propyl-2-[(2'-(tetrazol-5-yl)biphen-4-yl)-methyl]-1(2H)-isoquinolinone

Utilizing the general procedure outlined in 1(c), the product of 2(f) was deprotected to give the title compound as a foam. $^1$H-NMR ($CDCl_3$): 0.79 (d, 6H, J=6.7 Hz), 0.97 (t, 3H, J=7.0 Hz), 4.90 (s, 2H), 5.28 (s, 2H), 6.38 (s, 1H), 6.97 (m, 4H), 7.12-7.24 (m, 6H), 7.32-7.48 (m, 3H), 7.52 (t, 1H, J=7.7 Hz), 7.85 (bs, 1H), 8.07 (s, 1H).

## FORMULATION EXAMPLES

### Typical Pharmaceutical Compositions Containing a Compound of the Invention

A: Dry Filled Capsules Containing 50 mg of Active Ingredient Per Capsule

| Ingredient | Amount per capsule (mg) |
|---|---|
| Compound 1 | 50 |
| Lactose | 149 |
| Magnesium stearate | 1 |
| Capsule (size No. 1) | 200 |

Compound 1 can be reduced to a No. 60 powder and the lactose and magnesium stearate can then be passed through a No. 60 blotting cloth onto the powder. The combined ingredients can then be mixed for about 10 minutes and filled into a No. 1 dry gelatin capsule.

B: Tablet

A typical tablet would contain Compound 1 (25 mg), pregelatinized starch USP (82 mg), microcrystaline cellulose (82 mg) and magnesium stearate (1 mg).

C: Combination Tablet

A typical combination tablet would contain, for example, a diuretic such as hydrochlorothiazide and consist of Compound 1 (7.5 mg), hydrochlorothiazide (50 mg) pregelatinized starch USP (82 mg), microcrystalline cellulose (82 mg) and magnesium stearate (1 mg).

D: Suppository

Typical suppository formulations for rectal administration can contain Compound 1 (1-25 mg), butylated hydroxyanisole (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or

a diuretic and/or an angiotensin converting enzyme and/or a calcium channel blocker in pharmaceutically effective amounts as described, for example, in C above.

E: Injection

A typical injectable formulation would contain Compound 1 (5.42 mg), sodium phosphate dibasic anhydrous (11.4 mg) benzyl alcohol (0.01 ml) and water for injection (1.0 ml). Such an injectable formulation can also include a pharmaceutically effective amount of another active ingredient such as another antihypertensive and/or a diuretic and/or an angiotensin converting enzyme inhibitor and/or a calcium channel blocker.

**Claims**

1. A compound of the Formula (I)

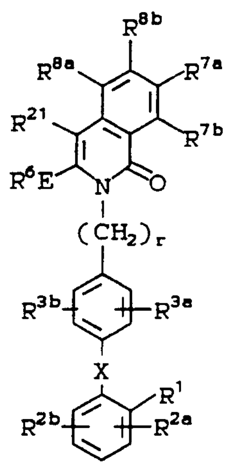

(I)

or a pharmaceutically acceptable salt thereof wherein:

$R^1$ is

(a) $-CO_2R^4$,
(b) $-SO_3R^5$,
(c) $-NHSO_2R^{22}$,
(d) $-PO(OR^5)_2$,
(e) $-SO_2-NH-R^{22}$,
(f)

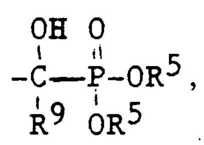

(g)

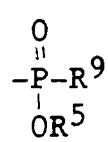

(h) $-SO_2NH-CO-R^{22}$,
(i) $-CH_2SO_2NH-CO-R^{22}$,
(j) $-CONH-SO_2R^{22}$,
(k) $-CH_2CONH-SO_2R^{22}$,

(l) $-NHSO_2NHCO-R^{22}$,
(m) $-NHCONHSO_2-R^{22}$,
(n)

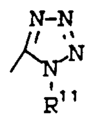 or 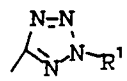,

(o)

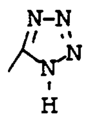 ,

(p)

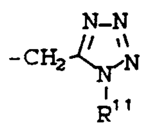 or 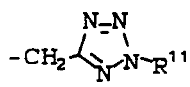,

(q)

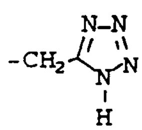 ,

(r)

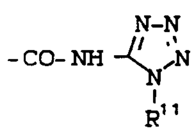 or 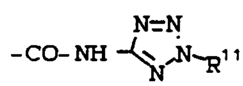,

(s)

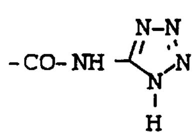 ,

(t) $-CONHNSO_2CF_3$ ,
(u) $-SO_2NH-CN$,
(v)

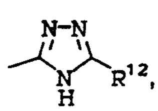,

(w)

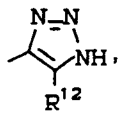

(x) $-PO(OR^5)(OR^4)$,
(y) $-SO_2NHCONR^4R^{22}$, or
(z) $-CH_2SO_2NHR^{22}$;

wherein heteroaryl is an unsubstituted, monosubstituted or disubstituted five or six membered aromatic ring which contains from 1 to 3 heteroatoms selected from the group consisting of O, N or S and wherein the substituents are members selected from the group consisting of -OH, -SH, $-C_1-C_4$-alkyl, $-C_1-C_4$-alkoxy, $-CF_3$, Cl, Br, F, I, $-NO_2$, $-CO_2H$, $-CO_2-(C_1-C_4$-alkyl), $-NH_2$, $-NH(C_1-C_4$-alkyl) and $-N(C_1-C_4$-alkyl$)_2$;

$R^{2a}$ and $R^{2b}$ are each independently
(a) H,
(b) Cl, Br, I, or F,
(c) $NO_2$,
(d) $NH_2$,
(e) $C_1-C_4$-alkylamino,
(f) di($C_1-C_4$-alkyl)amino,
(g) $SO_2NHR^9$,
(h) $CF_3$,
(i) $C_1-C_6$-alkyl,
(j) $C_1-C_6$-alkoxy,
(k) $C_1-C_6$-alkyl-S-,
(l) $C_2-C_6$-alkenyl,
(m) $C_2-C_6$-alkynyl;
(n) aryl,
(o) aryl($C_1-C_4$-alkyl), or
(p) $C_3-C_7$-cycloalkyl;

$R^{3a}$ is
(a) H,
(b) Cl, Br, I, or F,
(c) $C_1-C_6$-alkyl,
(d) $C_1-C_6$-alkoxy, or
(e) $C_1-C_6$-alkoxyalkyl;

$R^{3b}$ is
(a) H,
(b) Cl, Br, I, or F,
(c) $NO_2$,
(d) $C_1-C_6$-alkyl,
(e) $C_1-C_6$-acyloxy,
(f) $C_3-C_7$-cycloalkyl,
(g) $C_1-C_6$-alkoxy,
(h) $-NHSO_2R^4$,
(i) hydroxy($C_1-C_4$-alkyl),
(j) aryl($C_1-C_4$-alkyl),
(k) $C_1-C_4$-alkylthio,
(l) $C_1-C_4$-alkyl sulfinyl,
(m) $C_1-C_4$-alkyl sulfonyl,
(n) $NH_2$,
(o) $C_1-C_4$-alkylamino,
(p) di($C_1-C_4$-alkyl)amino,
(q) fluoro-$C_1-C_4$-alkyl-,

(r) $-SO_2-NHR^9$,
(s) aryl,
(t) furyl,
(u) $CF_3$,
(v) $C_2$-$C_6$-alkenyl, or
(w) $C_2$-$C_6$-alkynyl;

wherein aryl is phenyl or naphthyl, or a substituted phenyl or naphthyl with one or two substituents selected from the group consisting of Cl, Br, I, F, $N(R^4)_2$, $CO_2R^4$, $C_1$-$C_4$-alkyl, $C_1$-$C_4$-alkoxy, $NO_2$, $CF_3$, $C_1$-$C_4$-alkylthio, OH, or $SO_x(C_1$-$C_4$-alkyl);

$R^4$ is H, aryl, $C_1$-$C_6$ alkyl, or substituted $C_1$-$C_6$ alkyl in which substituent is aryl or heteroaryl as defined hereinabove;

$R^{4a}$ is aryl, $C_1$-$C_6$-alkyl or a substituted $C_1$-$C_6$-alkyl with an aryl substituent;

$R^5$ is H,

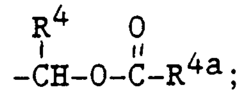

provided that when $R^4$ is H, $R^5$ is not H;

E is a single bond, $-NR^{13}(CH_2)_s-$, $S(O)_x(CH_2)_s-$ where s is 0 to 5, or CO-;

$R^6$ is

(a) aryl, or a substituted aryl with 1 or 2 substituents selected from the group consisting of Cl, Br, I, F, -O-$C_1$-$C_4$-alkyl, $C_1$-$C_4$-alkyl, $-NO_2$, $-CF_3$, $-SO_2NR^9R^{10}$, -S-$C_1$-$C_4$-alkyl, -OH, $-NH_2$, $C_3$-$C_7$-cycloalkyl, or $C_3$-$C_{10}$-alkenyl,
(b) $C_1$-$C_6$-alkyl, $C_2$-$C_5$-alkenyl or $C_2$-$C_5$-alkynyl, or a substituted $C_1$-$C_6$-alkyl, a substituted $C_2$-$C_5$-alkenyl or a substitued $C_2$-$C_5$-alkynyl, in which the substituent is selected from the group consisting of aryl, $C_3$-$C_7$-cycloalkyl, Cl, Br, I, F, $CF_3$, $CF_2CF_3$, $-NH_2$, $-NH(C_1$-$C_4$-alkyl), $-OR^4$ $-N(C_1$-$C_4$-alkyl$)_2$, $-NH-SO_2R^4$, $-COOR^4$, or $-SO_2NHR^9$,
(c) an unsubstituted, monosubstituted or disubstituted heteroaromatic 5 or 6 membered cyclic ring which contains one to three members selected from the group consisting of N, O, S, and wherein the substituents are members selected from the group consisting of -OH, -SH, $C_1$-$C_4$-alkyl, $C_1$-$C_4$-alkoxy, $-CF_3$, Cl, Br, I, F, or $NO_2$
(d) $C_3$-$C_7$-cycloalkyl,
(e) perfluoro-$C_1$-$C_4$-alkyl, or
(f) H;

$R^{7a}$, $R^{7b}$, $R^{8a}$ and $R^{8b}$ are independently

(a) H,
(b) $C_1$-$C_8$-alkyl or a substituted $C_1$-$C_8$-alkyl with a substituent selected from the group consisting of -OH, -guanidino, $C_1$-$C_4$-alkoxy, $-N(R^4)_2$, $COOR^4$, $-CON(R^4)_2$, $-O-COR^4$, -aryl, -heteroaryl, $-S(O)_x-R^{22}$, -tetrazol-5-yl, $-CONHSO_2R^{22}$, $-SO_2NH$-heteroaryl, $-SO_2NHCOR^{22}$, $-PO(OR^4)_2$, $-PO(OR^4)R^9$, $-SO_2NH-CN$, $-NR^{10}COOR^{22}$, $-(CH_2)_{1-4}R^4$, $-CO-R^4$, -CO-heteroaryl, $-NR^4CONR^4R^{22}$, $-NR^4COR^{22}$,

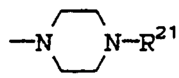, 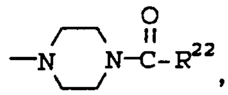,

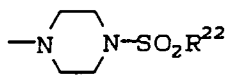 and 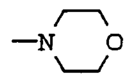 ,

(c) $-C_3-C_7$-cycloalkyl,
(d) aryl-$C_1-C_6$alkyl in which the aryl group is unsubstituted, mono or disubstituted with V or W,
(e) aryl or substituted aryl in which the substituents are V or W,
(f) Cl, Br, I, or F,
(g) $-OR^{22a}$,
(h) $-C_1-C_4$-perfluoroalkyl,
(i) $-S(O)_x-R^{22}$,
(j) $-COOR^4$,
(k) $-SO_3H$,
(l) $-NR^4R^{22}$,
(m) $-NR^{22a}COR^{22}$,
(n) $-NR^{22a}COOR^{22}$,
(o) $-SO_2NR^4R^9$,
(p) $-NO_2$,
(q) $-N(R^{22a})SO_2R^{22}$,
(r) $-NR^{22a}CONR^4R^{22}$,
(s)

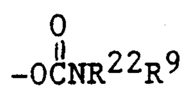,

(t) $-NHSO_2R^{22}$,
(u) $-SO_2NH$-heteroaryl,
(v) $-SO_2NHCOR^{22}$,
(w) $-CONHSO_2R^{22}$,
(z) $-PO(OR^4)_2$,
(y) $-PO(OR^4)R^4$,
(z) -tetrazol-5-yl,
(aa) -CONH(tetrazol-5-yl),
(bb) $-COR^{22a}$,
(cc) $-SO_2NHCN$
(dd) $-NR^{22a}SO_2NR^4R^{22}$,
(ee) $-NR^{22a}SO_2OR^{22}$
(ff) $-CONR^4R^{22}$,
(gg)

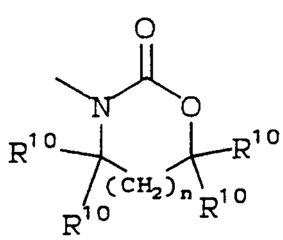

where n = 0 or 1,

(hh)

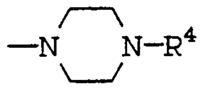 ,

(ii)

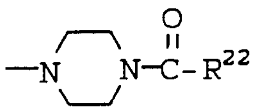 ,

(jj)

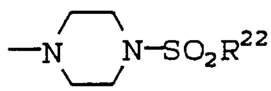 ,

(kk)

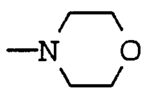 ,

(ll) heteroaryl as defined hereinabove, or

(mm)

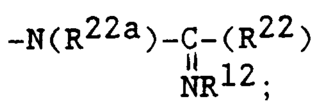 ;

V and W are independently:

(a) hydrogen,
(b) $C_1$-$C_5$-alkoxy,
(c) $C_1$-$C_5$-alkyl,
(d) hydroxy,
(e) $C_1$-$C_5$-alkyl-$S(O)_x$-,
(f) CN,
(g) $NO_2$,
(h) $N(R^4)_2$,
(i) $CON(R^4)_2$,
(j) $CO_2R^4$,
(k) $COR^4$,
(l) $CF_3$,
(m) Cl, Br, I, or F,
(n) hydroxy-$C_1$-$C_5$-alkyl,
(o) $C_1$-$C_5$-alkylthio,
(p) $-SO_2NR^9R^{10}$,
(q) $C_3$-$C_7$-cycloalkyl, or
(r) $C_2$-$C_{10}$-alkenyl;

$R^9$ is H, $C_1$-$C_5$-alkyl, aryl or arylmethyl;
$R^{10}$ is H, $C_1$-$C_4$-alkyl;
$R^{11}$ is H, $C_1$-$C_6$-alkyl, $C_1$-$C_4$-alkenyl, $C_1$-$C_4$-alkoxy alkyl, or

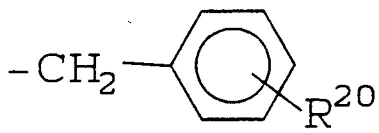

;

$R^{12}$ is -CN, $-NO_2$, $-CF_3$ or $-CO_2R^4$;
$R^{13}$ is H, ($C_1$-$C_4$-alkyl)CO-, $C_1$-$C_6$-alkyl, allyl, $C_3$-$C_6$-cycloalkyl, aryl or arylmethyl;
$R^{14}$ is H, $C_1$-$C_8$-alkyl, $C_1$-$C_8$-perfluoroalkyl, $C_3$-$C_6$-cycloalkyl, aryl or arylmethyl;
$R^{15}$ is H, $C_1$-$C_6$-alkyl;
$R^{16}$ is H, $C_1$-$C_6$-alkyl, $C_3$-$C_6$-cycloalkyl, aryl or arylmethyl;
$R^{17}$ is $-NR^9R^{10}$, $-OR^{10}$, $-NHCONH_2$, $-NHCSNH_2$,

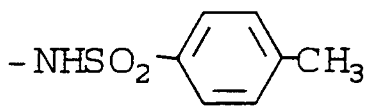

or

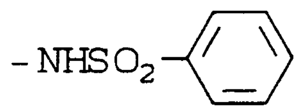

;

$R^{18}$ and $R^{19}$ are independently $C_1$-$C_4$-alkyl or taken together are $-(CH_2)_q-$ where q is 2 or 3;
$R^{20}$ is H, $-NO_2$, $-NH_2$, -OH or $-OCH_3$;
$R^{21}$ is

(a) H,
(b) Cl, F, Br or I,
(c) aryl as defined hereinabove,
(d) heteroaryl as defined hereinabove, or
(e) $C_1$-$C_4$-alkyl or a substituted $C_1$-$C_4$-alkyl with a substituent selected from the group consisting of aryl, heteroaryl, -OH, $-NH_2$, -NH($C_1$-$C_4$-alkyl),-N($C_1$-$C_4$-alkyl)$_2$, $-CO_2R^{4a}$, Cl, Br, F, I, or $-CF_3$;

$R^{22}$ is

(a) aryl,
(b) heteroaryl,
(c) $C_3$-$C_7$-cycloalkyl,
(d) $C_1$-$C_6$-alkyl or a substituted $C_1$-$C_6$-alkyl with one or two substituents selected from the group consisting of aryl, heteroaryl, -OH, -SH, $C_1$-$C_4$-alkyl, -O($C_1$-$C_4$-alkyl), -S($C_1$-$C_4$-alkyl), $-CF_3$, Cl, Br, F, I, $-NO_2$, $-CO_2H$, $CO_2$-($C_1$-$C_4$-alkyl), $-NH_2$, -NH($C_1$-$C_4$-alkyl), -N($C_1$-$C_4$-alkyl)$_2$, $-PO_3H_2$, -PO(OH)(O-$C_1$-$C_4$-alkyl), $-PO(OR^4)R^9$, morpholinyl, $-SO_xR^4$, piperazinyl-4-$COR^{22}$ or $C_1$-$C_4$alkylpiperazinyl, or
(e) perfluoro-$C_1$-$C_4$-alkyl;

$R^{22a}$ is

(a) hydrogen,
(b) aryl as defined hereinabove,
(c) heteroaryl as defined hereinabove,
(d) $C_3$-$C_7$-cycloalkyl,
(e) $C_1$-$C_6$-alkyl or a substituted $C_1$-$C_6$-alkyl with a substituent selected from the group consisting of aryl, heteroaryl, -OH, -SH, $C_1$-$C_4$-alkyl, -O($C_1$-$C_4$-alkyl), -S($C_1$-$C_4$-alkyl), $-CF_3$, Cl, Br, F, I, $-NO_2$, $-CO_2H$, $CO_2$-($C_1$-$C_4$-alkyl), $-NH_2$, -NH($C_1$-$C_4$-alkyl), -N($C_1$-$C_4$-alkyl)$_2$,

$-PO_3H_2$, $-PO(OH)(O-C_1-C_4\text{-alkyl})$, $-PO(OR^4)R^9$, morpholinyl, $-SO_xR^4$, piperazinyl-4-$COR^{22}$ or $C_1$-$C_4$ alkylpiperazinyl,
(f) perfluoro-$C_1$-$C_4$-alkyl, or
(g) $CH_2CH_2(C_2\text{-}C_4\text{-alkenyl})$;

X is

(a) a carbon-carbon single bond,
(b) -CO-,
(c) -O-,
(d) -S-,
(e)

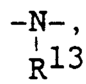

(f)

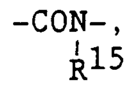

(g)

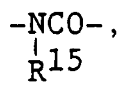

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,
(p) $-CH=CH-$,
(q) $-CF=CF-$,
(r) $-CH=CF-$,
(s) $-CF=CH-$,
(t) $-CH_2CH_2-$,
(u) $-CF_2CF_2-$,
(v)

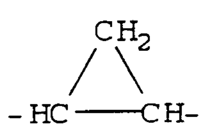

or

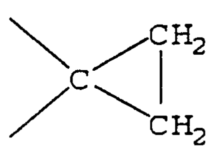

(w)

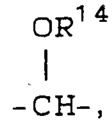,

(x)

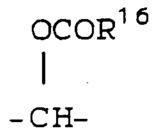

(y)

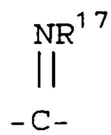,

or

(z)

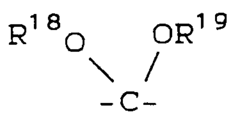;

r is 1 or 2, and

x is 0 to 2.

**2.** A compound of Claim 1 wherein:

$R^1$ is

(a) $-CO_2R^4$,

(b)

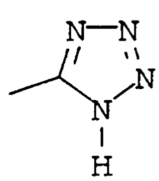,

(c) $-NH-SO_2R^{22}$;

(d) $-SO_2NH$-heteroaryl,

(e) $-CH_2SO_2NH$-heteroaryl,

(f) $-SO_2NH-CO-R^{22}$,

(g) $-CH_2SO_2NH-CO-R^{22}$,

(h) $-CONH-SO_2R^{22}$,

(i) $-CH_2CONH-SO_2R^{22}$,

(j) $-NHSO_2NHCO-R^{22}$,
(k) $-NHCONHSO_2-R^{22}$, or
(l) $-SO_2NHCN$;

$R^{2a}$ is H;

$R^{2b}$ is H, F, Cl, $CF_3$, $NO_2$, $C_1$-$C_6$-alkyl, $C_2$-$C_6$-alkenyl, $C_2$-$C_6$-alkynyl, or aryl;

$R^{3a}$ is H;

$R^{3b}$ is H, F, Cl, $CF_3$, $NO_2$, $C_1$-$C_4$-alkyl, $C_2$-$C_4$-alkenyl, $C_2$-$C_4$-alkynyl, $C_5$-$C_6$-cycloalkyl, $-COOCH_3$, $-COOC_2H_5$, $-SO_2-CH_3$, $NH_2$, $-N(C_1\text{-}C_4\text{-alkyl})_2$ or $-NH-SO_2CH_3$;

E is a single bond, -O- or -S-;

$R^6$ is

(a) $C_1$-$C_5$ alkyl or a substituted $C_1$-$C_5$ alkyl with a substituent selected from the group consisting of $C_3$-$C_5$-cycloalkyl, Cl, $CF_3$, $CCl_3$, $-O-CH_3$, $-OC_2H_5$, $-S-CH_3$, $-S-C_2H_5$, phenyl, or F;
(b) $C_2$-$C_5$-alkenyl or $C_2$-$C_5$-alkynyl; or,
(c) $C_3$-$C_5$-cycloalkyl;

$R^{7a}$, $R^{7b}$, $R^{8a}$ and $R^{8b}$ are independently

(a) H,
(b) $C_1$-$C_8$-alkyl or a substituted $C_1$-$C_8$-alkyl with a COOR, $OCOR^{4a}$, OH, aryl, $-(CH_2)_{1-4}R^4$, $-CO-R^4$, -CO-heteroaryl,

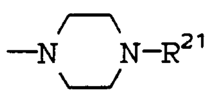, 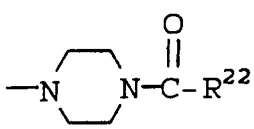,

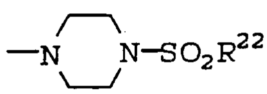 or 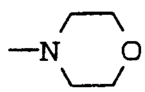

substituent,
(c) $OR^{22a}$,
(d) $-NO_2$,
(e)

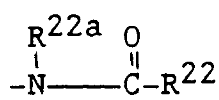,

(f) $-CONR^4R^{22}$,
(g)

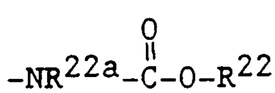,

(h) $-NR^4R^{22}$,
(i) Cl, F, or Br,
(j) $-CF_3$,
(k) $-CO_2R^4$,
(l) -CO-aryl,
(m) $-S(O)_x-R^{22}$,
(n) $-SO_2-NR^4R^9$,
(o) $-N(R^{22a})SO_2R^{22}$,
(p) aryl,
(q) heteroaryl,
(r) $-N(R^{22a})CONR^4R^{22}$,

(s) $-N(R^{22a})SO_2N(R^4)R^{22}$,
(t) $-N(R^{22a})SO_2OR^{22}$,
(u)

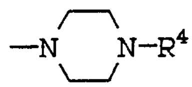,

(v)

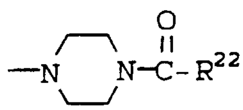,

(w)

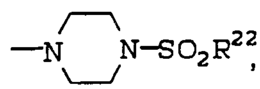,

or
(x)

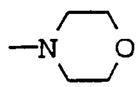;

$R^{21}$ is H, F, or Cl;
X is a single bond; and
r is one.

**3.** A compound of Claim 2 wherein:
$R^1$ is
(a) $-CO_2R^4$,
(b)

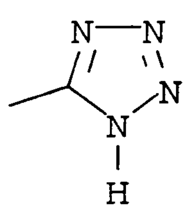,

(c) $-NH-SO_2-R^{22}$,
(d) $-SO_2NH$-heteroaryl,
(e) $-SO_2NH-CO-R^{22}$,
(f) $-CONH-SO_2R^{22}$, or
(e) $-SO_2NHCN$;
E is a single bond;
$R^{2b}$ and $R^{3b}$ independently are H, $-C_1-C_4$-alkyl, $-C_2-C_4$-alkenyl, $-C_2-C_4$-alkynyl, -Cl,

-F, $NO_2$, or $CF_3$;

$R^6$ is -$C_1$-$C_4$-alkyl, -cyclopropyl, -$CH_2CH_2CH_2CF_3$, $CH_2CH_2CF_3$, -$C_2$-$C_5$-alkenyl, -$CH_2OCH_3$ or -cyclopropyl; and

$R^{7a}$, $R^{7b}$, $R^{8a}$ and $R^{8b}$ are each independently H, -$C_1$-$C_4$-alkyl, -$NO_2$, -$NR^4R^{22}$, -$OCH_3$, -$N(R^{22a})$-$COOR^{22}$, -Cl, -$CH_2COOR^{4a}$, -$S(O)_x$-$R^{22}$, alkyl, $N(R^{22a})CON(R^4)R^{22}$, $CH_2OCO(C_1$-$C_4$-alkyl), $N(R^{22a})COR^{22}$, -F, -$CH_2Ph$, -$CONR^4R^{22}$, $CO_2R^4$, aryl, heteroaryl, -$C_1$-$C_4$-alkyl-heteroaryl as defined

hereinabove,

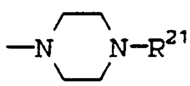, 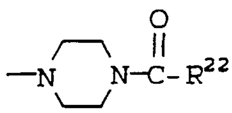, 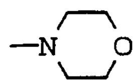,

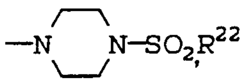 or 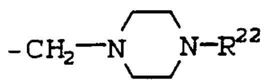.

**4.** A compound of Claim 3 of the Formula (II)

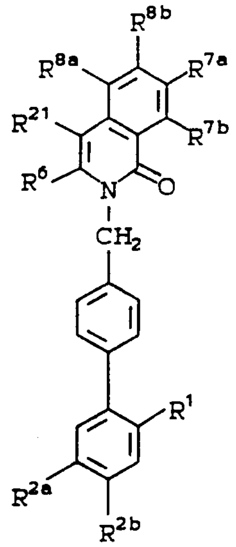

(II)

wherein:

$R^1$ is

(a) -$CO_2R^4$,

(b)

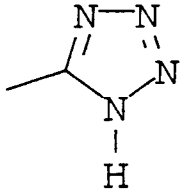,

(c) -$SO_2NHCOR^{22}$,

(d) $-CONHSO_2R^{22}$,
(e) $-NHSO_2R^{22}$, or
(f) $-SO_2NHCN$;

$R^{2a}$ and $R^{2b}$ are independently hydrogen or $C_1$-$C_4$ alkyl;

$R^6$ is

(a) $-C_1$-$C_4$ alkyl,
(b) $-CH_2CH_2CH_2CF_3$,
(c) $-CH_2CH_2CF_3$,
(d) $-CH_2OCH_3$, or
(e) cyclopropyl;

$R^{7a}$ is

(a) hydrogen,
(b) $C_1$-$C_4$ alkyl,
(c) $-NR^4R^{22}$,
(d) $-NR^{22a}CONR^4R^{22}$,
(e) $-NR^{22a}COR^{22}$, or
(f) $-NR^{22a}CO_2R^{22}$;
(g) aryl as defined hereinabove,
(h) heteroaryl as defined hereinabove,
(i)

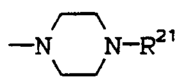

,

(j)

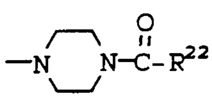

,

(k)

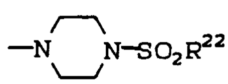

,

(l)

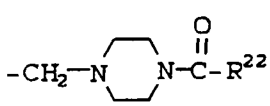

,

(m)

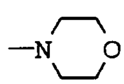

,

or
(n)

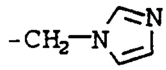 ;

$R^{7b}$ is
- (a) hydrogen
- (b) $C_1$-$C_4$ alkyl,
- (c) F, or
- (d) $CO_2R^4$;

$R^{8a}$ is
- (a) hydrogen,
- (b) $C_1$-$C_4$ alkyl,
- (c) F;

$R^{8b}$ is
- (a) hydrogen,
- (b) $C_1$-$C_4$ alkyl,
- (c) $-CO_2R^4$; and

$R^{21}$ is hydrogen or F.

**5.** A compound of Claim 4 selected from the group:
(1) 7-(N-methyl-N-isobutyloxycarbonyl)amino-3-propyl-2-[(2'-(tetrazole-5-yl)biphen-4-yl)methyl]-1(2H)-isoquinolinone,
(2) 7-(N-benzyl-N-isobutyloxycarbonyl)-amino-3-propyl-2-[(2'-(tetrazol-5-yl)biphen-4-yl)-methyl]-1-(2H)-isoquinolinone, and
(3) 3-propyl-2-[(2'-(tetrazol-5-yl)biphen-4-yl)methyl]-1(2H)-isoquinoline.

**6.** A pharmaceutical formulation for the treatment of hypertension and congestive heart failure comprising a pharmaceutically acceptable carrier and an effective antihypertensive amount of the compound of Claim 1.

**7.** The formulation of Claim 6 which includes an antihypertensive or a diuretic or an angiotensin converting enzyme or a calcium channel blocker which are members selected from the group consisting of: amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methylclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, verapamil, as well as admixtures and combinations thereof.

**8.** A pharmaceutical formulation useful in the treatment of hypertension which comprises a pharmaceutically acceptable carrier; a pharmaceutically effective amount of an antihypertensive or a diuretic or a converting enzyme inhibitor or a calcium channel blocker; and, a pharmaceutically effective amount of a compound of Claim 1; and, the pharmaceutically acceptable salts thereof wherein said antihypertensive said angiotensin converting enzyme inhibitor, and said channel blocker are members of the group consisting of amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, verapamil, as well as admixtures and combinations thereof.

**9.** The use of a compound of Claim 1 for the manufacture of a medicament for treating hypertension and congestive heart failure.

European Patent Office

# EUROPEAN SEARCH REPORT

Application Number

EP 92 20 0563

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 253 310 (E.I. DU PONT DE NEMOURS AND COMPANY)<br>* claims *<br>--- | 1-10 | C07D217/24<br>C07D401/10<br>C07F9/62<br>A61K31/47<br>A61K31/675 |
| A | JOURNAL OF MEDICINAL CHEMISTRY.<br>vol. 29, no. 10, October 1986, WASHINGTON US<br>pages 1953 - 1961;<br>SYLVESTER KLUTCHKO ET AL: 'Synthesis of novel angiotensin converting enzyme inhibitor Quinapril and related compounds.A divergence of structure-activity relationships for non-sulfhydryl and sulfhydryl types'<br>* the whole document *<br>--- | 1-10 | |
| A | EP-A-0 411 766 (MERCK AND CO.)<br>* claims *<br>--- | 1-10 | |
| A | EP-A-0 400 974 (MERCK AND CO.)<br>* claims *<br>----- | 1-10 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 19 MAY 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document